# EUROPEAN PATENT SPECIFICATION

(11) **EP 4 380 461 B1**
(45) Date of publication and mention of the grant of the patent: **23.09.2026**
(21) Application number: 22757960.4
(22) Date of filing: 29.07.2022
(51) Int. Cl.: A61B 10/00, A61F 5/451

(54) **URINE AND BODY LIQUID SAMPLE COLLECTION SYSTEM**
URIN- UND KÖRPERFLÜSSIGKEITSPROBENSAMMELSYSTEM
SYSTÈME DE COLLECTE D'ÉCHANTILLON D'URINE ET DE LIQUIDES CORPORELS

(30) Priority: 05.08.2021 NL 1044111
(43) Date of publication of application: 12.06.2024
(73) Proprietor: Capturin Holding BV, 5056 SM Berkel-Enschot (NL); Scint B.V., 5056 SM Berkel-Enschot (NL)
(72) Inventor: SCHILTHUIZEN, Stephanus, 5056 SG Berkel-Enschot (NL); GOIJARTS, Gregorius, 5062 CJ Osterwijk (NL)
(74) Representative: Rüger Abel IP Legal Solutions GmbH
(86) International application number: PCT/EP2022/071440
(87) International publication number: WO 2023/012072

(56) References cited:
- WO-A1-2016/051385
- WO-A1-2018/139988
- US-A- 5 203 327
- US-A- 5 957 904
- US-A1- 2004 176 731
- US-A1- 2007 270 716
- US-A1- 2015 051 510
- US-A1- 2021 196 501

## Description

### BACKGROUND AND STATE OF THE ART

This invention relates generally to the field of body fluid sampling devices for diagnostic and treatment purposes to be able to monitor parameters and biomarkers in the collected urine and other collected body fluids of patients, such as perspiration (sweat), interstitial fluid and blood. More specifically, the invention relates to the safe, secure and comfortable collection of urine samples from persons who are not able to produce an urine sample themselves on command in a predetermined manner in a container or in another device which can collect the urine. Urine is a body liquid, which contains many ions, analytes, electrolytes and molecules which are biomarkers for the functioning of organs, body liquids, tissues and the body itself. The invention also relates to collection of other fluids such as perspiration liquid or sweat, wound fluids, blood and interstitial fluids for diagnostic purposes and tissue recovery.

US 2004/0176731 A1 discloses a system for collecting samples pf urine or body fluids exuding from the skin of user. The system comprises a base part of a pliable material adapted to be draped on the skin of a user closely following the contours of a predetermined body part of the user. The base part has an inner side facing the skin of the user. Furthermore, the system comprises a urine or fluid collecting part provided on the inner side of the base part and having a capillary structure. This structure is adapted to receive and absorb urine or fluids excreted by the user. The base part comprises on its inner side at least an area defined by a barrier line or rim encircling at least a part of the urine or fluid collecting part exposed to the user. The barrier line or rim is adapted to sealingly engage the skin of the user, thereby preventing intrusion of any contaminations into the area surrounding the urine or fluid collecting part and leakage of urine or body fluids therefrom. Further prior art can be taken from US 2015/0051510 A1, US 5,957,904, WO 2018/139988 A1, US 5,203,327, US 2021/0196501 A1, US 2007/0270716 A1, WO 2016/051385 A1.

For diagnosis of a number of diseases and for monitoring of the functioning of organs, urine and other body fluid samples can be collected by medical personnel and be analyzed on presence and concentrations of ions, molecules and chemical compositions. The collection of a non-contaminated sample is therefore essential to be able to assess accurately the quality of the urine and to measure accurately the quantities of the ions and molecules, which act as a biomarker, and which should not elute (originate) from the collection device or material. Examples of these ions, electrolytes and molecules are for instance calcium, creatinine, phosphates etc. Calcium is a biomarker for the "English disease", or Rickets disease, in which too much Calcium is disposed of by the body, resulting in weaker bones and bow-legs. A higher value of creatinine can be an indicator of a failing kidney functioning. Varying values of potassium, especially lower values, can be indication of and or cause of metabolic problems, blood pressure and kidney clearance problems. Contamination of the urine samples can occur when the urine is mixed with fecal parts containing proteins, bacteria, fats and other elements, or when the skin releases bacteria and other substances into the urine sample.

Another important aspect of urine sampling collection is to be able to collect urine samples of persons who are not able to produce an urine sample on command themselves, e.g. who don't have a toilet or potty trainedness or cannot cooperate easily in a clean catch. These persons can either be very young, premature, dysmature or underdeveloped born babies who have to stay in special incubators in children departments in hospitals, or are born without complications but don't have toilet training till age of appr. 3 years, or are people which are physically or mentally disabled and of high age, who also can't produce an urine sample on command themselves.

One common method of medical diagnosis is urine analysis for diagnostic purposes. In most cases, an urine sample is easily obtained as the process is relatively simple and does not require any type of assistance from another person. But, in the case of a neonate as described above, the process is more difficult as a neonate has no control over its urinary functions. Moreover, a neonate cannot produce and collect an urine sample the way an older and potty trained child or an adult can. It will be appreciated that these same issues may also pertain to comatose adults, and nursing home patients. However, under certain circumstances, even older patients may be unable to assist in collecting a urine sample.

However, also the lying in a puddle of urine and excessive washing of the babies' skin can lead to skin irritation, rash, and other skin-related problems. Thus, also a need exists for an improved collection and testing method for newborn and premature babies that can help minimize skin-related issues arising from collection bags, apart from the main diagnoses purpose itself.

Currently collection of urine specimens from patients, and in particular neonatal, premature born infants, and mentally handicapped patients, often involves the use of polymer urine collection bags or cups or cotton balls and cotton gauzes. To be able to collect a specimen from an infant requires attachment of a urine collection bag or container to or near the infant. There are a number of problems associated with this approach. Often it is difficult to ensure that the bag remains attached to the infant, frequently resulting in leakage, and or contamination of the urine sample with feces. Often the medical personnel has to attach the urine collection bag a number of times, in most cases at least 3-4 times, before an urine sample can be collected and frequent sampling is not possible due to skin irritations and skin damages as well as reliability problems. The adhesive materials used to attach the collection bag often cause namely skin irritation and skin damages, particularly if frequent specimens, samples are required, in combination with the higher stiffness and low ability of the collection bag material and glue layer to follow closely and without shear forces the contours of the skin in the collection area. This can be a significant problem in very premature infants, who have very fragile skin and skin vulnerable to irritations and allergic reactions and need frequent sampling to measure various biomarkers in the urine. Also the bags are very stiff and rigid on the part where the adhesive is applied to the skin and are not able to follow the anatomic contour of the baby precisely, causing discomfort, leakage and or contamination. With older, more active infants it can often be difficult to ensure that the collection bag remains attached to the child. Also in a number of cases the urine is contaminated with fecal parts (proteins. bacteria, fats, white blood cells etc.).

The other main state of the art solution is the use of cotton gauzes, balls or vilt type of urine absorbers. These are put into the small diapers of the premature or dysmature born infants, which are then attached to the body of the infants. When the infant produces urine it will be collected by the cotton gauze, ball or vilt absorption elements. The disadvantages of these methods are that cotton absorbers or gauzes consist of hollow, cotton based fibers, which contain in most cases inorganic and organic, parts, analytes and ions, which influence the diagnostic measurement of the urine of the infant and secondly that feces parts can easily contaminate the urine sample with bacteria, proteins etc. The vilt type used for absorbing tasks, often contains elements which influence the actual measurement. Both cotton and vilt absorbers are placed within the diaper and can thus be contaminated with feces, fecal parts, skin bacteria, proteins, fats while they're not blocked by any kind of barrier.

Both types of present collection systems result in multiple attempts and applications before a clean urine sample can be collected. This leads to unwanted skin irritations and damages, unnecessary disturbing, discomforting and interruption of the resting and sleeping period of the premature born infants. It causes stress and influences growth and strengthening of the infants. It would be preferred to have a solution which can be applied numerous times without problems, making frequent, regular and reliable monitoring of biomarkers possible.

The neonatal care personnel lacks therefore an adequate means to collect safely urine, with a high comfort for the infants and with a high reliability and accuracy of acquiring a clean urine sample without contamination of feces. There's a need for a method and device, which is able to do this safely, quickly and with a high comfort level for the nursing personnel.

Secondly there's not an adequate, reliable and safe method available for collection of urine samples of patients which are not able to produce easily an urine sample on command. A safe, reliable and effective solution to collect the urine sample automatically is very needed also for other user groups: ill patients, incontinent elderly and disabled persons, non potty trained children etc.

### Detailed description of the invention

The invention describes a system to collect safely, without instructions and without on command handlings of the patient, without contamination and in a comfortable way and collect urine of patients, which are not able to produce an urine sample themselves on command or who are not easily able to do so for various other reasons. The invention is set out in the appended set of claims. Key and essential element of the invention is the use of non-hollow synthetic polymer yarns, fibers or polymeric parts or elements, which have capillary uptake capacity in the cavities between the material parts and which will after special cleaning treatment with chemicals and or demineralized water not elute ions, salts, molecules and other micro components originating from the fiber material itself in the urine sample which is being collected in the fibers. The used types and structures of capillary materials and their cleaning methods to prepare them for the use as sampling material will ensure that the maximum elution of analytes, electrolytes and molecules from the capillary material in the liquid which forms sample to be analyzed is not above 0,1 mmol/L. This applies also for the adhesion from analytes, electrolytes, molecules which are inherent present in the liquid to the capillary material. The elution and adhesion levels of electrolytes, analytes, ions and molecules which have to be measured, will be for the applied and prepared materials in the invention not be above 0,1 mmol/L. The combination of non-hollow, massive yarns consisting of non-hollow, massive fiber elements or filaments will prevent that water from finishing processes will stick to, and in the yarns or fibers, and will avoid therefore the elution of Calcium, Potassium, Sodium and other ions and molecules such as creatinine and phosphates from the fiber surface into the urine sample.

An important key aspect or element of the invention is to collect the urine with part of a device, which consists largely of synthetic yarns, each made of one or more filaments or comparable capillary materials. These yarns, preferably in a woven, knitted or non woven, sealed or braided capillary constructions are able to absorb and transport urine from the point where the baby, child or adults excretes from the orifice of the urethra to the initial contact point of the device via the capillary cavities and small spaces between the material filaments and fibers to the points, area where the urine sample is stored in the device temporarily, before it can be collected for further lab analysis. The non-hollow filaments or fibers will not retain liquid itself, but transport the liquid via capillary effects in the open space between yarns, fibers, fiber filaments, from the point of collection to the area and volume of storage. Capillary effects can realized via the use of multifilament polymer yarns and fibers in a fine knitted, woven or non-woven structure. Also a possible embodiment is the use of natural, synthetic, polymeric or inorganic foams and powder or sintered structures with connected capillary cavities and spaces around the foam, powder, sintered material. The urine which the patient produces will drop on one of the ends or unexposed parts of the capillary structure and will be transported into the open spaces of the capillary material till it saturates and is filled with the required urine sample volume. The size of the pores, open spaces, inter yarns spaces, inter fibers spaces, inter fiber filaments spaces should be small enough that the surface tension between liquid and fiber material has such a value that the material will enable transport of liquids in the cavities, open spaces or "rise" of liquids. The surface tension should therefore not be too high to block the transport The surface tension of the capillary material can be modulated with special treatments in the finishing and cleaning process, by use of specific coatings, additives etc. The capillary spaces formed between yarns may be termed as macrocapillary and the capillary spaces present or formed between fibers, filaments in yarns may be termed as microcapillary. Both types of space, cavities can contain liquid after uptake of these in the material.

Essential in all possible embodiments of the invention is that the device consists of two main parts: the base part which can be draped, wrapped or put on the skin or on or around anatomic parts in the vicinity of male penis or female urethra, and which is closely following the contours of the anatomy of the body part and the skin it is put on and which is connected to the skin via sticky polymer adhesive lines and a second capillary part wrapped. This capillary part is packed in a polymer film casing, packaging, wrapping and this capillary part is able to absorb and contain urine or body fluids via an exposed end and not wrapped section which is directly in the vicinity of the urethra in case of a female patient and the tip of the penis in case of a male patient. The base part can have any kind of shape, depending in some embodiment partly on the sex of the patient, and can an oval, a circular, a round, an ellipse shape with one or more smaller extensions, butterfly shaped, hexagonal, octagonal, 3D cylinder shaped, 3D shaft shaped, 3D balloon shaped and any kind of regular or irregular 2D and or 3D shape. Depending on the shape and size of the base part the device is suitable for female, male or both female and male patients. The capillary part can be extending in any kind of direction from the base part or be incorporated in the base part and have a certain length so that it can be draped on the leg or lower or upper belly of the patient and it can have such a length that the end section of it can be visible outside the diaper so that urine collection in the capillary part can be noticed directly by medical personnel or consumer users. The capillary part can also be within the circumference of the base part in a folded, non folded and wrapped and protected condition apart from the end near the urethra or penis which collects the urine. In all embodiments the capillary part has an unexposed receiving part for urine which is positioned within the surface area or volume of the base part of the device near the urethra orifice of the patient and is attached to the base part.

Another key element of the invention is mentioned briefly in the previous paragraph and is the use of a capillary structure of knitted, woven, braided, or non woven multifilament yarns, fibers, monofilament yarns or fibers or combinations of these yarns, fibers to take up and absorb the urine from the urethra orifice and to store it in the capillary structures and open spaces between fibers and filaments. Essential is that there is a distance and surface area between the receiving end of the capillary structure and the point where the urine can maximally flow to. This distance should be large enough to prevent quick drying of the urine from the exposed end, and should be in combination with width and thickness of the capillary structures large enough to take up the requested sample volume. Another key element is that the capillary structure consisting of woven, knitted, non woven or open foam structures has a certain length and surface area and volume that is able to absorb a certain quantity of urine and which has such a length that at least the end part of it can be positioned outside the diaper of child or incontinent person, so that the nurse can notice when it will be filled partly or completely satirized with urine. The capillary structure itself is covered with a transparent inert polymer silicone cover or other inert polymer, synthetic or natural, film, which can have the shape of a flat bag or sleeve with an opening at the side where the capillary material will be exposed with one or more extended ends for urine collection. This will be described later in detail. This enables visual identification of filling of it with the coloured urine. These polymer films and covers, wrappings, or small bags will not influence the measurement of analytes, electrolytes and molecules in such a way that they are above the desired accuracy, threshold level of 0,1 mmol/L. They're preferably made of inert polymers such as silicone printed or extruded film, polyethylene etc.

Another key element of the invention is that the end section of the capillary structure has one or more sections or end parts, which are not covered by a silicone or water-resistant polymer film and are able to absorb the urine coming from urethra or penis directly and transport it further in the rest of the capillary structure. These can be one, two or more parts, which are integral part of the rest of the textile or non-textile based capillary structure, so that urine can be dropped onto these ends and be sucked up rapidly and transported further in the capillary structure. The use of multiple ends has two main reasons. First of all, the capacity to start collecting and transporting the urine is higher when there're 2 or more end parts or sections for the initial receiving of the urine droplets which are produced by the patient. Secondly the use of more than one end part, unexposed receiving part, preferably 2, 3 or more, connected each with a part of folded or single sheet capillary material will guarantee that there will be no blockage of urine collection and transport due to impregnation of the silicone film wrapping on the capillary material or due to other reasons.

The capillary structure can be a single sheet or single part with any kind of shape, crosse section and dimension or can consist of 2, 3, 4 or more parts of one integral piece of material folded to a long and thin package or they can be folded, enrolled in any kind of shape which can be covered in a polymer, PUR, silicone film, or other polymer film or foil, to prevent de-hydration and contamination of the sample. It can also have any kind of shape provided the fact that the used material has sufficient capillary capacity and the characteristics and geometries that enable uptake and inter capillary absorption of urine and other liquids without absorption in the actual capillary materials. The liquid will be drawn into the capillary cavities, small spaces between the polymer material(s), small spaces between filaments of used polymer fibers, small spaces between the fibers itself created by the production process (knitting weaving, braiding, non-woven melting etc.).

Another key element of the invention is to create one or more physical and mechanical barriers between the point where feces is excreted, being the anus, and the area where the orifice of the urethra or penis is and where urine will be excreted and be collected by the capillary part of the device. These barrier lines or rims have a certain height, width and surface area and will prevent the entry of fecal part from the anus of the patient to the inner area where the urine sample collection will be performed. These barrier lines or rims can have the shape of a raised area, and or can have the shape of a special type line, film with adhesive surface characteristics, and or can have the shape of a raised area made of adhesive, flexible and cushioning material, and or can consist of a line or area of material which can be sealed to skin by pulling and or pushing away the air underneath it, can be a small or wider rim which is pushed to skin with a slighter higher pressure by a vacuum sealing, by an additional elastic strap around one or more parents of the body of the wearer, by combination of elastic strap and an adhesive element. These barrier lines are preferably extruded or 2D, or 3D printed, or silk screen printed onto a textile or a coated textile material, on the skin side of the base part of the device and will have a high adhesion to the skin and an easy peel-off characteristic. They can have a low, moderate, or high compressibility and resilience, enabling in the latter cases a higher comfort and ensuring sealing during movements and manual handlings of care tasks. They can have any kind of height and width and a resulting sufficient adhesion surface area to the skin, forming with this the actual barrier for urine leakage from the inside urine collection area of the device to the part of the skin which is not covered by the device and a barrier for the entry of fecal and other contamination parts in the urine collection section. They can be printed with a 2D or 3D extrusion print nozzle, with a silk screen printing method, with inkjet printing or any kind of printing or application method. The barrier lines can be composed of a mixture of two kind of polymer components: a sticky component and a non sticky component. Preferably the mixture is consisting of more than 50% of a sticky component and less then 50% non sticky component The polymer can be of liquid silicone types in two different compositions, which are mixed in a mixer and applied on the surface with one of the mentioned application, printing techniques. The silicone mixture can have components with a higher hardness and components with a lower, softer hardness. Also use of gas insertion is possible to raise the compressibility and softness. The silicone can be of the type Silpuran of Wacker silicone or of another type. The combination of the more fluid sticky version and the other more rigid and solid type with higher viscosity with lower resulting stickiness gives the optimum mixture combination. The polymer can also be of another type, provided the fact that it retains its sticky character after application which can be used to get adhesion and sticking effect to the skin. Possible other polymers are Polyurethane, natural sticky polymers, skin friendly sticky hotmelts etc.

To enhance the attachment to the skin the inner side of the device can have beside the printed, sticky barrier lines, a number of printed non sticky polymer dots such as silicone dots with the same height as the sticky barrier lines, rims with a cylindrical or spherical shape, with a flat or rounded contact surface, positioned in the surface area between the outer and inner barrier lines, rims. These dots with the same diameter of varying diameters will create a vacuum effect and pull the skin extra firmly to the device, enhancing the attachment to the skin.

The second possible barrier element which is structural integrated in the design and construction is the use of a watertight coating and or use of watertight materials on the inside and or outside of the device, so that the feces, fecal parts, can also not get near the urine collection and absorption point via other routes, apart from the shortest route between the anus and the area of the device near the orifice of urethra.

Another key element of the invention is that the end of the capillary structure has one or more soft, high comfort ends, which are not covered by a polymer or silicone film and are able to absorb the urine coming from urethra or penis directly and transport it further in the capillary structure. These can be one, two or more parts with a highly hydrophilic surface and urine and water absorbing capillary structure.

The capillary structure can be single sheeted, a double folded sheet, a triple folded, a spacer fabric material with 3 layers (top layer, intermediate layer and bottom layer) etc. or can be a material with a porous structure with interconnected pores and cavities or non or partially interconnected cavities in any kind of shape, as long as it is able to absorb, and more specifically contain urine in its capillary cavities, and in the open spaces between fibers, material parts, filaments etc., The material can be textile, made of polymer, synthetic non-hollow, massive fibers, can be a paper, cellulose based, can be a natural or synthetic foam material with open and or closed cellular structure, can be an inorganic ceramic material, sintered porous ceramic or polymeric material or can consist of multiple capillary small polymer tubes etc. Also the capillary structure can consist of combinations of materials. Also the use of individual not sintered or not to another melted powder parts is possible, contained in a specific construction and with a receiving end which is one integral part of powders.

Another key element of the invention is the use of multifilament yarns, fibers for the woven, knitted , braided or non woven or any type of textile structure of the capillary system. These fibers can have a smaller number of filaments (preferably less than 200 filaments per fiber, preferably 10-50 filaments per fiber, per yarn, and a decitex thickness of preferably 10-50, but not limited to this mentioned number of filaments and decitex thickness) to reduce the possibility to capture and mechanically bond ions, salt and molecules between the filaments after the urine comes in contact with these fibers. Also filaments with other and various cross sections can be used in the fibers, such as round, oval, bilobal, trilobal, square, rectangular, triangular, hexagonal, octagonal, irregular etc. In a preferred version of this material selection the fibers are made of smooth, non texturized polymer filaments, with a regular, smooth surface, without many possible mechanical anchoring points for the ions, salts and molecules. Also a mixture of multifilament smooth and or texturized fibers and monofilament fibers is a possible embodiment The textile capillary structures with these or other characteristics can be made of pigmented or non-pigmented fibers, which are only industrially washed with demineralized of de-ionized water of regular cleaning water and then thermically fixated. In case of use of regular non de-ionized water in washing and fixation of these materials, additional pre-cleaning and cleaning steps are necessary with chemicals and other solution to remove calcium and other elements before the final use.

Another key element of the invention is the use of contoured, shaped materials of the device which can easily adapt to the contours of the body around the anus, urethra, penis, belly etc. and drape itself on the skin etc. Textiles or textile based drape able materials are a good solution for this, more in particular if the base shape of the device has rounded edges or radii in the corners, or has one, two or more rounded protruding ends, which are outside the baseshape of the main part of the material and device and which can curve, drape on and around the skin and body on the points further away from the central area where the device of the invention will be positioned being the urethra orifice area.

The base part of the invention will have in a number of embodiments a shape with two or more rounded corner parts, protruding ends which are smaller in dimension can easily follow the contours of the anatomy and the skin can be easily fastened to the skin, whereas the center part to which this two or more rounded ends are connected will have the shape of an ellipse, a circle or any other kind of regular or irregular shape. A possible embodiment of the invention is that it has four rounded, curved ends all connected to a central ellipse shaped part, creating a shape which looks partly like the baseshape of a butterfly, or can have any other shape, such as oval, ellipse shaped, circular, rectangular with small or large radii in the corners, hexagonal, octagonal, irregular shaped etc. The basepart can even have a longer length and larger shape protruding and touching at the suprapubic area to be able to stimulate the urine production with incorporated phase change materials in the basepart, which will give a cooling effect to the belly of the child patient to stimulate urine production (quick wee effect via phase change materials). A cooling effect on this area will in many cases stimulate the urine sample production. The body heat of the patient is transferred to the basepart with the integrated phase change materials and these will be subsequently transfer these in lowering of th temperature of the device with several degrees. The phase change materials can be incorporated, integrated in the fibers, yarns of the applied textile or in the coatings applied on these materials.

All baseparts can have one or more "pull-tabs" integrated, which are not glued to skin and which can easily be grasped by the fingers to remove the device gently and completely from the patient's skin.

Essential is in all embodiments that the device consists of two main parts: the base part which is connected to the skin around the genital, pubic area and above this area via sticky polymer adhesive lines and a capillary part wrapped in polymer film, which is able to absorb urine or body fluids via an exposed end and not wrapped section which is directly in the vicinity of the urethra in case of a female patient and the tip of the penis in case of a male patient.

The capillary part will absorb the body fluid such as urine and perspiration, and is afterwards centrifugated in a laboratory to extract the body fluid such as urine and measure via photo spectrometry and or electrical detection methods ions, molecules, analytes and electrolyte presence and concentrations in the fluid such as urine.

Another key element is the preferable use of Polyester, Polyethylene, Polypropylene, Polyamide, Dyneema, HDPE, UHMPE, PTFE, Aramide, Twaron, hollow Viscose or any kind of synthetic fibers in the capillary structure and, or in the textile material of the base part of the device. These fibers can be monofilament fibers or multifilament fibers. Also natural fibers can be used provided the fact that they're cleaned and rinsed properly and are resistant against acidic and alkalic urine.

Another possible embodiment of the invention is that the capillary part is made of a 3-dimensinal knitted or woven spacer fabric with use of multifilament fibers, yarns in the upper surface, and or inner area and or bottom surface. This ensures a higher absorption and transport capacity of the capillary part.

Another possible embodiment of the invention is that the capillary part is made of synthetic or natural open cellular or partly closed cellular foam material, such polyolefin foam types, PUR foam types and other synthetic foam types with interconnected cavities, a porous ceramic material, a combination of a polymer and ceramic material in a sponge like structure.

Another possible embodiment of the invention is that the capillary part is made of a larger number of capillary tubes with a small diameter which are able to absorb the urine.

Another key element of the invention is that the capillary, urine absorbing materials consist of hollow and or non hollow fibers, yarns which have been cleaned with specific washing or cleaning processes to avoid elution of elements present within the fibers or outside on the fiber surface in the urine and by this influencing the actual measurement of diagnostic parameters in the urine. The cleaning procedures of the material can include one or more of the following cleaning steps in any kind of order:
- Pre cleaning with a surfactant solution (soap, alkali based solution, de-ionized water, acid solution or other type). Mechanical stirring during pre-cleaning.
- Rinsing, cleaning with demineralized or de-ionized water
- Cleaning with an acidic solution made of acid and deionized, demineralized water (citric acid, acetic acid, peracetic acid, nitric acid etc.
- Rinsing and cleaning can be done at 20 degrees Celsius or at 23 Degrees Celsius or at room temperature or at any elevated temperature above room temperature or at any kind of temperature, but preferably above room temperature.
- Treatment in an ion exchanger solution which exchanges ions such Ca2+ with Na+ or any kind of ion exchange.
- All pre-cleaning, cleaning, rinsing and ion exchanger solutions, liquids can be stirred automatically with a mechanic, electric or magnetic device or element or by hand or combinations thereof.
- All pre-cleaning, cleaning, rinsing and ion exchanger solutions, liquids can be pulled through the capillary material via a vacuum system, in which the vacuum is releases and the mentioned liquids flow through the capillary material and drop below in a containment space, area, container and are by release of this vacuum pulled away from the textiles,
- Wringing, mechanical pressing on the materials to press out water and cleaning residues
- Drying on air or with electric or mechanical dryer
- Sterilization steps: with ethylene oxide, autoclave or steam sterilization with deionized water, microwave, gamma sterilization etc.
- All these or parts of these cleaning steps can be one in mechanical automatic constructions and specially designed machinery.

Another possible embodiment of the invention is that the capillary, urine or body liquid absorbing materials are produced, made with a so called Avivage finishing consisting of lubricants emulsions made of deionized water solutions to minimize the elution of Ca2+ and other ions from the so called Avivage, the outside wrapping of the fibers, yarns to the urine sample.

Another possible embodiment of the invention is that the capillary structure is made of a non-finished polymer textile fabric, which is then pre-cleaned and cleaned with one or more of the cleaning steps described above to prepare it for use in the device.

Another possible embodiment of the invention is that the capillary structure consists of a 2D or 3D printed or sintered polymer or ceramic foam structure, in which small droplets of any kind of polymer material are extruded into a partly open capillary structure or are sintered with a laser from initial powder parts or any other sintering process resulting in a porous structure.

Another possible embodiment of the invention is that the capillary structure consists of cellulose material or paper based material made with de-ionized water. The cellulose or paper based structure sucks up the urine in its structure and will release it after centrifugation. The use of paper which is produced with deionized water will guarantee a high recovery and a low adhesion of elements which have to be measured in the urine.

Another possible element of an embodiment of the invention is that the end part, section of the capillary structure or the complete capillary structure can be made more acidic with an acidic coating and or integrated solid acid oxide or salt parts to be able to raise the pH value of the urine sample which is collected from the patient after dissolving water. In an example of an embodiment an acidic coating is applied on and or in the capillary structure. Liquid or solid salt parts which can lower the pH value of the urine sample as soon as they dissolve from the capillary structure in the urine can be integrated via a spray process on the capillary material (liquid, dissolved salts in a solution), an electrostatic process (solid salts), a paint or print process or a plasma coating process. In case of an electrostatic process the salt powder parts will be dispersed randomly and or in a pattern in the capillary structure and will be connected to fibers, yarns or material of the capillary structure via melting or mechanical adhesion.

Another possible embodiment of the device is that the capillary structure has a limited length and then it only acts as a transport medium to absorb and transport the fluid or urine comfortably and easily without contamination and let it subsequently drop in the collection bag of polymer film of the device. This solution can guarantee limited elution of analytes from the capillary material in the urine, in which these analytes have to measured, such as Ca, Na etc. Also this solution can prevent or limit the amount of absorption or adhesion of analytes which are present in the urine to the capillary material, which could influence the measurement of analytes, electrolytes in the diagnostic sample.

.Another possible embodiment of the capillary materials is that the capillary textile structure is coated with a nanocoating of inorganic or organic composition or combinations thereof which prevents adhesion of Ca-salts, Ca-ions and Ca-molecules and improves capillary function and hydrophilic character of the material. It is also possible to apply a thin silicone spray coating or finish on the fibers to enable fast uptake and transport urine within the capillary structure and to prevent adhesion of ions, salts and molecules to the textile structure.

Another possible embodiment of the capillary material is that the capillary textile structure is coated with specific molecules or chemical compositions which can be used to capture biomarkers in the shape of ions or molecules. These "capture" molecules will release the biomarker parts, ions and or molecules, in the actual diagnostic sampling and analysis process. The adhesion of the biomarker molecules to the capturing molecules on the capillary materials can be measured after separation in the urine which is centrifugated, or can be measured directly with optical detection methods, while they're still connected to the capillary materials or electrical or combinations of optical and electrical methods.

Another possible embodiment of the invention is that it is able to collect perspiration, sweat from the skin. The base part can be coated on the inside with a hydrophobic coating such as an inert silicone coating. The outer circumference is provided with an adhesive printed line. Within the inner area perspiration vapour will condensate from the skin of patient to liquid droplets and these perspiration droplets will be collected directly in the inner area. The inner area can be a single one or can be multiple in number, surrounded each by a printed, adhesive polymer line, to retain the perspiration within its inner area and to prevent permeation and evaporation of the perspiration. In a special embodiment of this perspiration collection version, the collection area(s) is covered at the outside with an inert polymer and along the circumference of the area all over the height, thickness of the cross section of a textile material with capillary function to create a liquid barrier in all directions, areas which will not take up the urine sample. The non coated inner surface of the collection area with capillary textile, foam, powder or other material, can than retain extra volume of perspiration liquid. Perspiration can be collected afterward in the laboratory with centrifugation or other techniques.

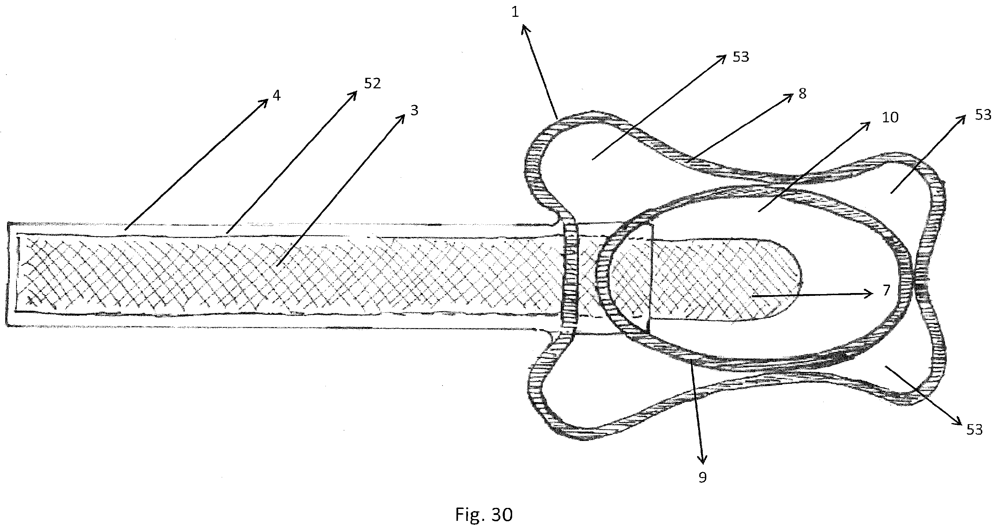

Another possible embodiment of the invention is a special version for collection and analysis of wound fluid samples and creation of drainage from the wound itself and collection of wound fluids. This version can have the same characteristics as the urine collection version. The receiving end(s) of the capillary part are positioned above the wound, whereas the sticky printed polymer barrier lines circle and outline around the wound area. The wound fluid is absorbed by the capillary part and depending on the purpose this can be analyzed for determination of tissue healing process or it is also done and repeated regularly for wound fluid drainage and faster recovery of tissue, absorbing fluids within the wound and transporting them into the capillary part This will keep the wound dryer and bacteria present in the wound fluid will be removed from the wound area. For this specific embodiment the size and shape of the basepart can be different Application of the adhesive barrier rims of printed polymers will also be present within the design, combined with a capillary part with protruding receiving end(s), This receiving end can be larger of size depending on the wound size, which also applies to the basepart dimension. Essential is that the sticky adhesive barrier line or rims can be positioned outside the wound periphery. The inner area of the basepart is also in this embodiment preferably coated with an inert polymer such as silicone to prevent leakage and sticking of this area to the wound area. This area can also be provided with thin grid like printed pattern of small non sticky silicone lines, which will also prevent the sticking of this area to the wound area, but also has sufficient capillary absorption area around them within the inner receiving area (see for designs and construction also the embodiments of fig. 30, 31, 32 and 33), to take up wound fluids and drain the wound to get it more dry to stimulate tissue recovery.

List **of figures:**
Fig. 1 shows a top view of an embodiment of the device with the capillary part under an angle
Fig. 2 shows a top view of an embodiment of the device with a fixation system for the urine receiving end of the capillary part
Fig. 3 shows a top view of an embodiment of the device with the capillary part parallel to the main, butterfly shaped part of the device.
Fig. 4 shows a top view of an embodiment of the device with the capillary part parallel to the main, butterfly shaped part of the device and with a fixation system for the urine receiving end of the capillary part.
Fig. 5 shows a side view and cross section of the device with the capillary part entering the butterfly from the outer, non skin side, with the fixation system on the inside.
Fig. 6 shows a side view and cross section of the device with the capillary part entering the butterfly from the outer, non skin side with a tape sealing at the outer side,
Fig. 7 shows a 3D view of the capillary part of the device in unfolded condition.
Fig. 8 shows a 3D view of the capillary part of the device in folded condition.
Fig. 9 shows a 3D view of the capillary part of the device in flat condition.
Fig. 10 shows a 3D view of the capillary part of the device in flat condition with the polymer wrapping.
Fig. 11 shows a cross section of the device with a capillary part 3 in the polymer film 4.
Fig. 12 shows a cross section of the device with a capillary part 3 in the polymer film 4 which act as a transport medium and is shorter in length than the actual polymer film wrapping.
Fig. 13 shows a side view and cross section of the device with the capillary part, partly folded and covered in a polymer, cup or bag shaped film.
Fig. 14 shows a cross section of the device out the capillary part with coating on the inner side and outer side.
Fig. 15 shows a cross section of the device out the capillary part with coating on the inner side.
Fig 16 show a top view of a possible embodiment of the device.
Fig. 17 shows a 3D view of the device mounted on a female baby.
Fig. 18 shows a 3D view of the device mounted on a male baby.
Fig 19 shows a 3D view of a possible embodiment of a male device.
Fig. 20 shows a 3D view of a possible embodiment of a male device.
Fig. 21 shows a cross section of a possible embodiment of the device.
Fig. 22 shows a 3D view of a possible embodiment of a male device.
Fig. 23 shows a 3D view of a possible embodiment of a device.
Fig. 24 shows a cross section of a possible embodiment of the device.
Fig. 25 shows a 3D view of a possible embodiment of a male device.
Fig. 26 shows a 3D view of a possible embodiment of a male device.
Fig 27 shows a 3D view of a possible embodiment of a female or male device.
Fig 28 shows a 3D view of a possible embodiment of a female or male device.
Fig 29 shows a 3D view of a possible embodiment of a female or male device in plano, flat production stage condition.
Fig 30 shows a 2D view of a possible embodiment of a female device made in one integral piece.
Fig 31 shows a 2D view of a possible embodiment of a device for collection of perspiration with a single, large collection area
Fig 32 shows a 2D cross section of a possible embodiment of a device for collection of perspiration.
Fig 33 shows a 2D view of a possible embodiment of a device for collection of perspiration with multiple collection areas.

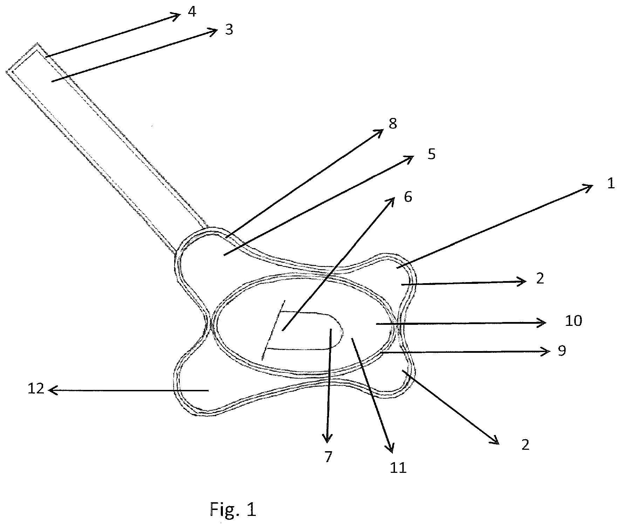

Fig. 1 shows a top view of the inner part, skin side part a possible embodiment of the device for female patients with the capillary part 3 under an angle with the actual main and base part of the device 1 and extending form the base part. This main part 1, which can be made of textile and or inert polymer film or combinations thereof, is bendable, drapeable and flexible and can adapt itself to the anatomy of the patient and follow the body contours. Also part 3 is flexible, bendable and can, because it is under an angle with the center line of the base part, be positioned and placed on the right or left leg of the patient, resulting in that a part of it will be visible outside the diaper that the patient is wearing, so that the medical personnel can see if the capillary part has absorbed urine of the patient Part 1 has one or more ends 2 and 12, which are smaller in dimension, rectangular, curved or otherwise shaped and integrally connected to the rest of part 1. These ends 2 and 12 can facilitate a better fitting to the body contour and enable fastening of the device to it Either the 2D shaped cutout, being the non existing material of part 1 between parts 2 or between parts 12 can be used to position the device just outside the anus of patient. Part 1 will therefore not be glued on the anus but glued around the anus and upwardly in the direction of the belly, Part 1 has at its circumference a sticky, raised line of printed compressible polymer 8 which acts as an adhesion line to mount and stick the device to the skin of the patient and act as a first barrier against feces,

The capillary part 3 is connected to part 1 via a slit 6 in part 1 and enters the device part 1 from the outer, non skin side. The capillary part 3 is covered in a polymer film sleeve or wrapping 4, which protects the urine or body fluids from dehydration and contamination before it is actually processed in the laboratory for diagnostic measurements. The capillary structure has a part 7, which is not covered with a polymer film, but which is protruding on the inner side of the device and is capable of absorbing the urine that the patient produces. This urine will be transported in the capillary structure of 3 upwardly. The rounded end 11 of the capillary part 7 can be a single end, a double, a triple etc., depending on the amount of folded or joint parts of the structure 3. Use of more than one non exposed, non covered parts as part 11 will enable faster absorption of urine and will ensure the fact that at least one of the folded parts of the capillary material will have a direct entry point for urine. The polymer coating, wrapping of the capillary part can have impact on the capillary transport capacity of the parts of the materials which are in contact with it. The rounded end is more comfortable for the patient, but can also be rectangular with roundings, radii in the corners or ellipse shaped. The part 1 has also two ends 5 which can be equal to the end part 2 or bigger in size and differ in shape. Between these two end parts 5 there is an open, partly round, cutout space which the nurse can use to position the device exactly at the right side of the anus and covering the urethra.

The angle between part 3 and part can be 15, 30, 45 degrees or any kind of preferred angle. The part 3 can be partly underneath end 5 or end part 12, so that it will be on the left or right leg of the patient, depending on the nursing practice. The main part 1 has an inner area 10, which is limited by a sticky, raised line, rim of polymer 9. This raised line, with any kind of height, can be extruded, 2D printed, or silk screen print or applied in any other matter and forms a barrier against feces and will keep the urine that the patient produces within the area 10. This area 10 is coated with an inert polymer, not able to absorb or elute ions and other molecules, elements influencing the measurement in the urine, which preferably is hydrophobic, but can also be hydrophilic and can be texturized to stimulate transport of urine to the capillary part. This ear 10 is sealed hermetically to the skin and will function as collection area for e.g. the urine, All the urine which the patient produces will thus drop on this area 10, be retained within this area and will flow consequently and also be dropped directly on the capillary end part 7. Also the urine which isn't dropped directly on part 7, will be absorbed by this capillary part 7 via the intermediate receiving surface, area part 10.

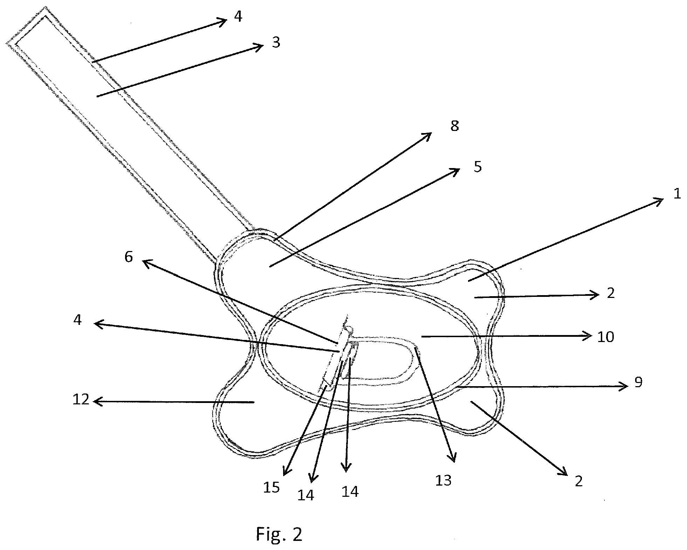

Fig. 2 shows a top view of the inner part, skin side part a possible embodiment of the device for female patients with the capillary part 3 under an angle with the actual main part of the device 1. This main part 1 is bendable, drapeable and if necessary flexible and can adapt itself to the anatomy of the patient and follow the body contours. Also this main part 1 can be partly elastic to increase the ability to fasten it to the skin of the patient. Also part 3 is flexible, bendable and can be put on the leg of the patient, with a part of it visible outside the diaper that the patient is wearing.

The capillary part 3 is connected to part 1 via a slit 6 and enters the device part 1 from the outer, non skin side. The capillary part 3 is covered in a polymer film sleeve, cartridge or wrapping 4. This polymer wrapping 4 enters the device via slit 6 and is glued with its outside, bottom part to the printed sticky polymer lines 14 and 15, just along the slit 6. The area on the inside of the device where the actual textile or foam based capillary part will be exposed to urine is printed with similar sticky lines 13 and 14 which enable to fasten the end part 7 with rounding 11 to it (see for these parts fig 1). This will ensure a mechanical fastening of part 4 including part 3 to the actual device 1, and will prevent leakage of collected urine to the bottom part of the device (the non-skin side, the outside when mounted on the patient). A possible embodiment is also that these printed lines 14 and 15 are printed on the backside, non skin side of the device and provide mechanical fastening of the capillary part here and prevent leakage.

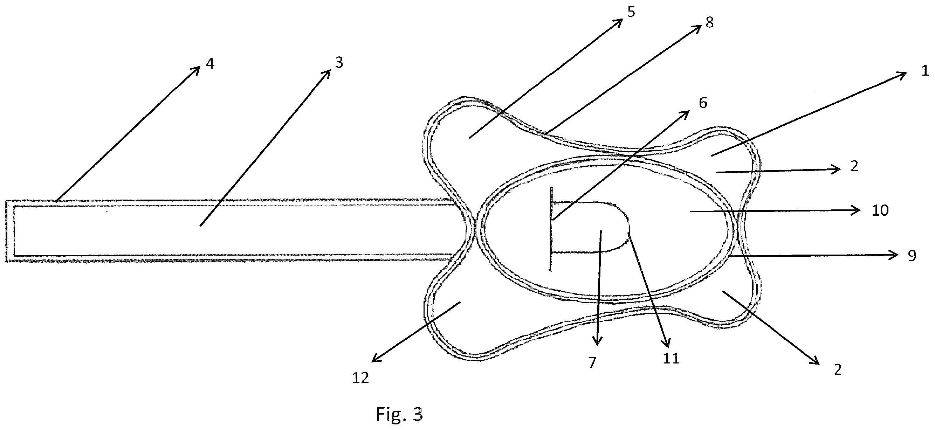

Fig. 3 shows a top view of the inner part, skin side part and possible embodiment of the device for female patients with the capillary part 3 parallel to and in line with the actual main part of the device 1. This main part 1 is bendable, drapeable and flexible and can adapt itself to the anatomy of the patient and follow the body contours. Also part 3 is flexible, bendable and can be put on the leg of the patient, with a part of it visible outside the diaper that the patient is wearing. Part 1 has one or more ends 2 which are smaller in dimension, rectangular, curved or otherwise shaped and integrally connected to the rest of part 1. These ends 2 can facilitate a better fitting to the body contour and enable fastening of the device to it Part 1 has at its circumference a sticky, raised line, rim of polymer 8 which acts as an adhesion line to mount and stick the device to the skin of the patient and act as a first barrier against feces.

The capillary part 3 is connected to part 1 via a slit 6 and enters the device part 1 from the outer, non skin side via this slit 6. The capillary part 3 is covered in a polymer film sleeve or wrapping 4, which protects the urine or body fluid from dehydration and contamination before it is actually processed in the laboratory for diagnostic measurements. The capillary structure has a part 7, which is not covered with a polymer film, but which is protruding on the inner side of the device and is capable of absorbing the urine that the patient produces. This urine will be transported in the capillary structure of 3. The rounded end 11 of the capillary part 7 can be a single end, a double, triple etc., depending on the amount of folded parts of the structure 3. The rounded end is more comfortable for the patient, but can also be rectangular with a rounding in the corners or ellipse shaped. The part 1 has also two ends 5 which can be equal to the end part 2 or bigger in size and differ in shape. Between these two end parts 5 is an open space, cut out area, with which the nurse can position the device exactly at the right side of the anus and covering the urethra.

The angle between part 3 and part 1 can be 30, 45 degrees or any kind of angle. The part 3 can be partly underneath end 5 or end part 12, so that it will be on the left or right leg of the patient, depending on the nursing practice or it can be parallel with the center line of main, base part 1. The main part 1 has an inner area 10, which is limited by a sticky, raised line of polymer 9. This raised line, which can be extruded, 2D printed, or silk screen print or applied in any other matter forms a barrier against feces, solid and liquid fecal parts and will keep the urine that the patient produces contained within the area 10. This also applies to raised polymer printed line 8. The polymer used for these raised lines can be any kind of polymer and preferably has a sticky character or a high adhesion capacity to the human skin. It is preferably compressible and flexible so that can easily adapt to the skin and follow the skin contours and irregularities closely. The polymer can be a synthetic or non-synthetic polymer, it can consist of one or more components. These components can be a sticky type of a polymer and a non sticky type of a polymer, mixed together in any kind of composition. The polymer can also have gas or air injected into it, to make it even more compressible, flexible and soft. Preferably the raised lines, rims 8 and 9 have the same height, but they can differ in height and width and composition of material. The raised lines, rims 8 and 9 can follow the outer contours of the complete device 1 and the inner part 10, but can also be at any specific area between these two mentioned lines or even inside the inner area 10, to ensure good adhesion to the skin of the patient. The number of printed lines can be one, 2, 3 or more. Also the area between the printed lines 8 and 9 can be provided with extra printed sticky polymer lines, rims or surfaces to enhance the attachment to the skin.

To enhance the attachment to the skin the inner side of the device can have beside the printed, sticky barrier lines 8 and 9, a number of printed non sticky polymer dots with the same height or somewhat smaller height with a flat or rounded skin contact surface, in the surface area between the barrier lines (not drawn in the figure). These dots with the same diameter of varying diameters will create a vacuum effect and pull the skin extra firmly to the device, enhancing the attachment to the skin.

The area, part 10 is coated with an inert polymer, which preferably is hydrophobic, but can also be hydrophilic or area. An example or possible embodiment is the use of a non sticky silicone finish or coating applied by any kind of process. Part 10 is an area which is watertight and can't absorb urine. All the urine which the patient produces will drop on this area 10 and directly on the capillary end part 7, Also the urine which isn't dropped directly on part 7, will be absorbed by this capillary part 7, due to the inability of area 10 to absorb urine. Part 1 can be one integral part with area 10. It can consist of woven, knitted or non woven synthetic or natural textile material. Part 10 can be made watertight and or hydrophobic via a polymer coating, such as a silicone, PUR, Polyacetate, ink coating, or via a plasma treatment. The area or part 10 can also be a separate watertight part in the shape of the desired area 10 which is glued, sealed or mechanically fasted to part 1.

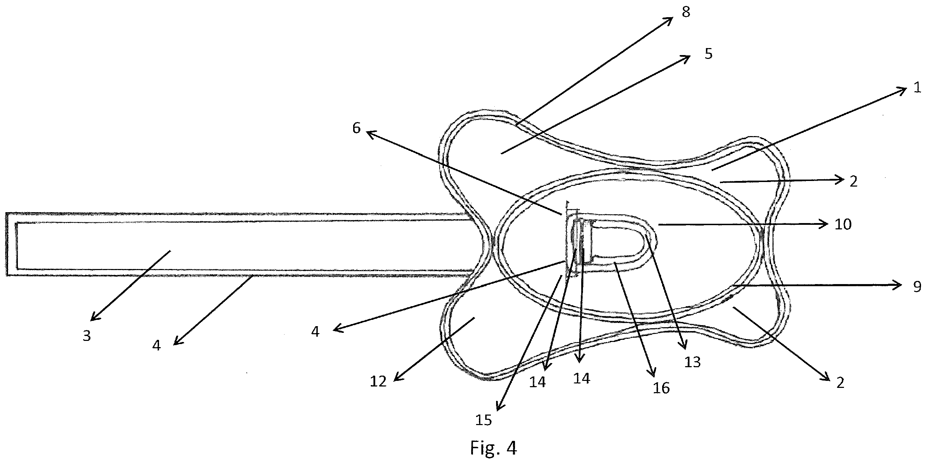

Fig. 4 shows a top view of the inner part, skin side part an another be possible embodiment of the device for female patients with the capillary part 3 parallel to and in line with the actual main part of the device 1. This main part 1 is bendable, drapeable and or flexible and can adapt itself to the anatomy of the patient and follow the body contours. Also part 3 is flexible, bendable and can put on the leg of the patient, with a part of it visible outside the diaper that the patient is wearing to make it visible when it is filling with the urine sample or other body liquid.

The capillary part 3 is connected to part 1 via a slit 6 and enters the device part 1 from the outer, the non skin side via this slit 6. The capillary part 3 is covered in, with a polymer film sleeve or wrapping 4. This wrapping can have the shape of a long bag and is preferably made of a transparent polymer, so that the professional medical user, van see when urine has been collected in the capillary part, This polymer wrapping 4 enters the device via slit 6 and is glued with its outside, bottom part to the printed, sticky polymer lines 14 and 15, just beside and along the slit 6. The area on the inside of the device where the actual textile or foam based capillary part will be exposed to urine is printed with similar sticky lines 13 and 14 which enable to fasten the end part 7 with rounding 11 to it (see for these parts fig 1). A second printed 16 or a third can be added to improve the fastening. Also the whole area underneath part 7 can be provided with a sticky polymer layer or a number of printed lines next to each other or close to each other. These measures will ensure a mechanical fastening of part 4 including part 3 to the actual device 1, will prevent leakage of collected urine to the bottom part of the device (the non-skin side, the outside when mounted on the patient). A possible embodiment is also that these printed lines 14 and 15 are printed on the backside, non skin side of the device and provide mechanical fastening of the capillary part here and prevent leakage.

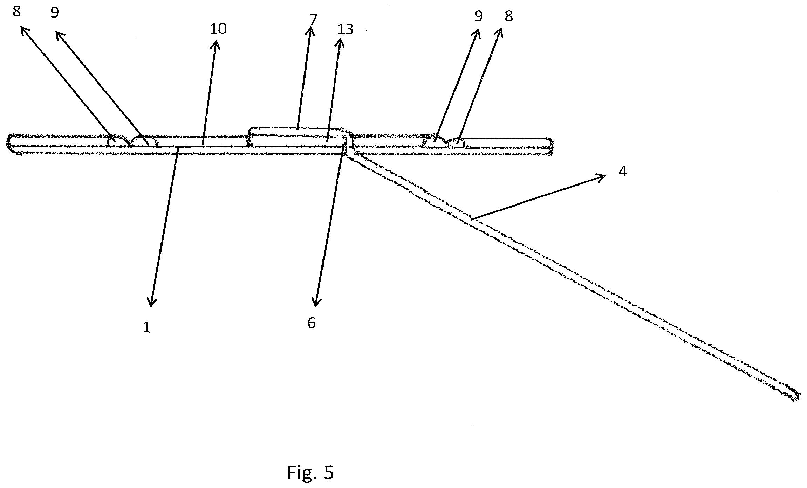

Fig. 5 shows a side view and cross section of the device with the capillary part 4 entering the butterfly from the outer, non skin side, with the fixation system 13 and other sticky polymer lines on the inside. Also visible in this side view and cross section are the printed polymer lines 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, outside. Part 7 which is the unexposed part of the capillary material 3 within part 4, is the part on which the patient drops his or her urine. The urine will be absorbed here and will transported in the capillary device material 3, till this is saturated with urine and or the urine production stops.

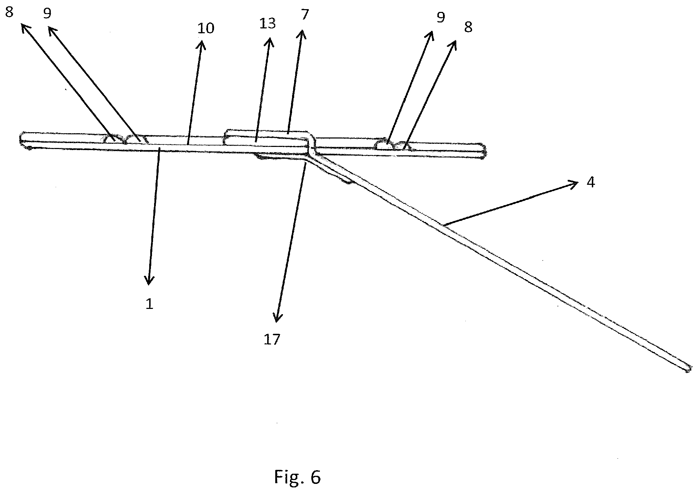

Fig. 6 shows a side view and cross section of the device with the capillary part entering the butterfly from the outer, non skin side with a tape sealing at the outer side. It shows the capillary part 4 entering the butterfly from the outer, non skin side, with the fixation system 13 and other sticky polymer lines on the inside of the device. In this embodiment the mechanical fastening and leakage prevention can be improved with a separate polymer tape 17 sealed on the backside, outer side of the device. This tape 17 covers the slit and partly the polymer film wrapping 4 of the capillary part 3 and sticks also to the material 10. This sealing 17 can also be a printed surface of polymer such as silicone or PUR, which seals off the slit 6. Also visible in this side view and cross section are the printed polymer lines, rims 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, outside. Part 7 which is the unexposed part of the capillary material 3 within part 4, is the part on which the patient drops his or her urine. The urine will be absorbed here and will transported in the capillary device material 3, till it is saturated with urine.

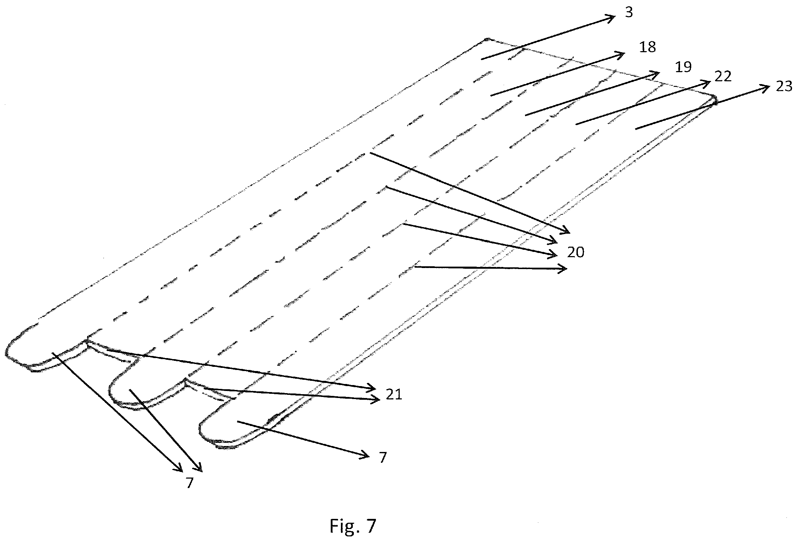

Fig. 7 shows a 3D view of the capillary part of the device. The capillary material 3 is shown here in non covered unfolded condition, without the polymer film wrapping 4. The capillary part 3 can consist of 1, 2, 3, 4 or more segments, all connected to each other and made of one integral piece of material. These parts can also be single, individual parts which are mechanically or sealed to each other, still enabling liquid uptake and capillary transport. These segments 18 19, 22, 23 can be folded along the lines 20 and can have 1, 2 or more rounded ends 7, which are protruding from the segments 18, 19, 22, 23.

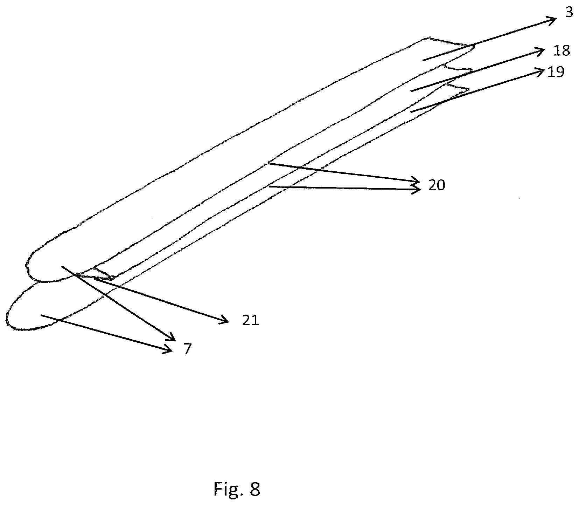

Fig. 8 shows a 3D view of the capillary part of the device. The capillary material 3 is shown here in non covered folded condition, without the polymer film wrapping 4. The capillary part 3 can consist of 1, 2, 3, 4 or more segments, all connected to each other and made of one integral piece of material. These segments 18, 19 etc. are folded along the lines 20 and can have 1, 2 or more rounded ends 7, which are protruding from the folded package of the segments 18, 19.

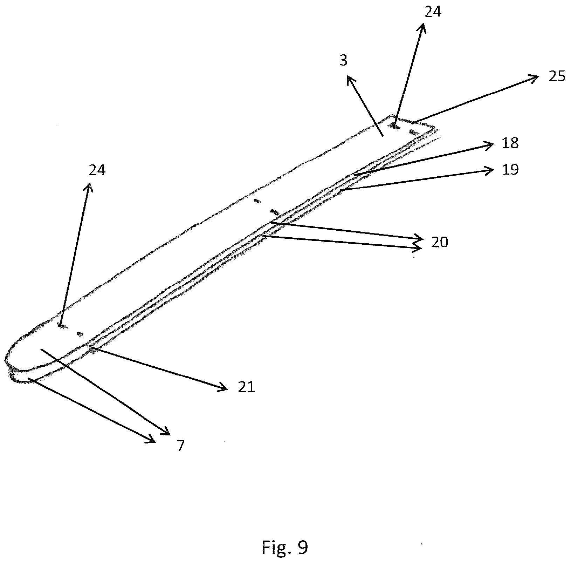

Fig. 9 shows a 3D view of the capillary part of the device in flat condition. The capillary material 3 is shown here in non covered folded, not in film packed condition, without the polymer film wrapping 4. The capillary part 3 can consist of 1,2, 3, 4 or more segments, all connected to each other and made of one integral piece of material. These segments 18, 19 etc. are folded along the lines 20 and can have 1, 2 or more rounded ends 7, which are protruding from the folded package of the segments 18, 19. In this embodiment the folded parts 3, 18, 19 are kept together via welding lines, points 24, or sealings lines, points 24. These welding points or lines 24, will not have the full width of one folded element 3, 18, 19, but just a small part of it, to guarantee the transport of urine and liquid past these points 24 from the entry point at 7 till the end part 25.

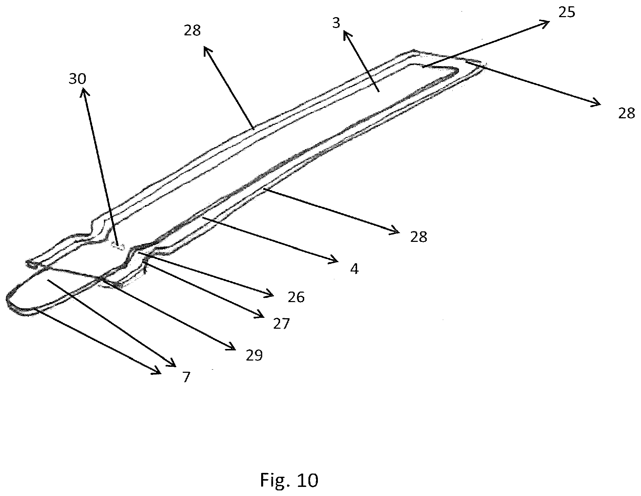

Fig. 10 shows a 3D view of the capillary part 3 of the device in flat, folded condition with polymer wrapping 4 largely covering it. The capillary material 3 is shown here in covered folded condition, with the polymer film wrapping 4. The capillary part 3 can consist of 1, 2, 3, 4 or more segments, preferably all connected to each other and made of one integral piece of material. They can also be separate parts provide the fact that they're closely packed together in the film wrapping 4, so that the capillary absorption can be transferred from one single part to another. These segments can have a reduction of area 27 on two sides, to enable easy tearing off. This restriction and reduction of area can also be present in the polymer film wrapping 27. The capillary material can be perforated partly in this section on position 30. This will enable easy tearing off after the sample has been collected. The polymer film wrapping 4 can consist of two layers of film which are glued or sealed together along the lines 28 which cover the three sides of the wrapping which has to be sealed. A possible embodiment is that the polymer film wrapping consists of one part which is folded along the line 28 and sealed or glued at the other side. The polymer film wrapping of the capillary part 3 can be a silicone film which is glued with a printed silicone lines along the lines 18. It can also be a silk screen printed polymer film which is printed on both sides of the capillary part 3 and which is sealed together along the lines 28 directly after printing. The polymer wrapping or packaging of the part 3 can also be a Polyurethane film, a Polyethylene, Polyester, acetate film or any kind of watertight, polymer film which doesn't elute ions and other analytes and elements after contact with urine. The wrapping can also be extrusion blown polymer film in one piece as a bag, an injection moulded bag etc.

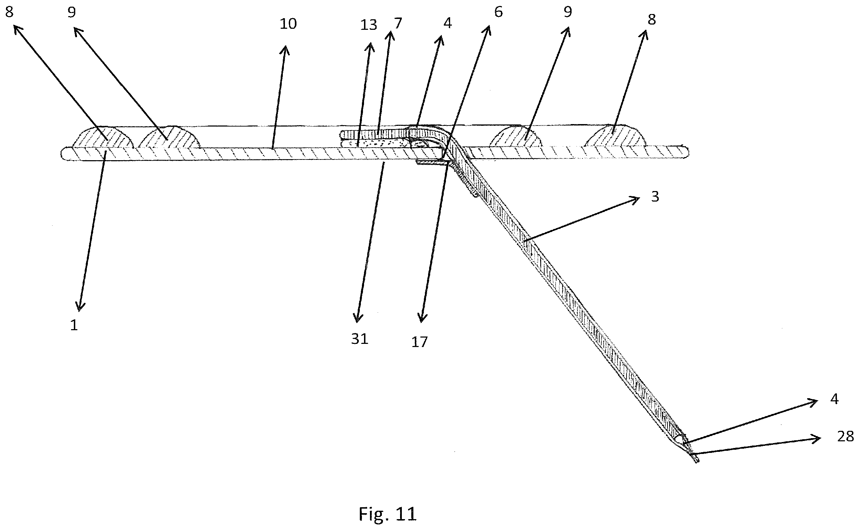

Fig. 11 shows a side view and cross section of the device with the capillary part entering the butterfly from the outer, non skin side with a tape sealing at the outer side. It shows the capillary part 4 entering the butterfly from the outer, non skin side, with the fixation system 13 and other sticky polymer lines on the inside of the device. In this embodiment the mechanical fastening and leakage prevention can be improved with a separate polymer tape 17 sealed on the backside, outer side of the device. This tape 17 covers the slit 6 from one side and partly the end of the polymer wrapping 4 of the capillary part 3 and sticks and is glued also to the material 10. This sealing 17 can also be a printed surface of polymer such as silicone or PUR, which seals off the slit 6. One can choose between either for only the printed lines or areas 13, or the tape sealing or printing 17 or for both solutions to guarantee the mechanical fastening of part 3, 4 to part 1 and the prevention of leakage of urine or fluid from the inner receiving side 10, and part 7 via the slit to the back side, the outer side 31 or leakage within the textile part 1. Also visible in this side view and cross section are the printed sticky polymer lines, rims 8 and 9 which enable fastening of the device to the skin of patient and form a double barrier to prevent entering of feces parts, which would contaminate the urine sample to be collected. Part 10 is the base material of the device which can be coated with a watertight, inert, polymer on the inside and if necessary, on the outside. Part 7 which is the unexposed part of the capillary material 3 within part 4, is the part on which the patient drops his or her urine. The urine will be absorbed here and will transported in the capillary device material 3, till it is saturated with urine.

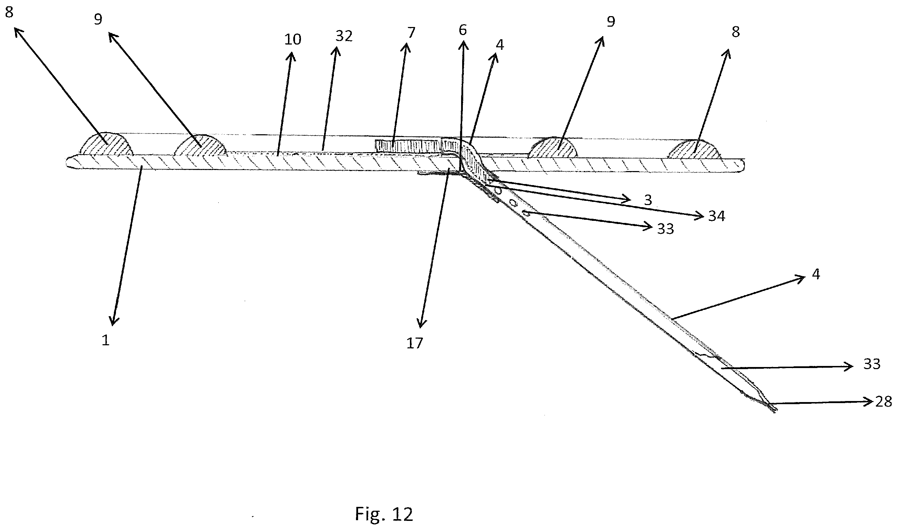

Fig. 12 shows a side view and cross section of the device with the capillary part entering the butterfly, or base part from the outer, non skin side with a tape sealing at the outer side. It shows the capillary part 3 covered in the film 4 entering the butterfly from the outer, non skin side, via slit 6 to the inner part 10 which can be covered with a hydrophobic silicone film or printed layer 32 in all embodiments. The capillary part 3 is in this embodiment shorter in the length than the polymer film wrapping 4, so that it acts purely as a transport medium. The liquid or urine 33 is absorbed at part 7, and flows into and enters part 3 further and the drips subsequently from the end 34 of part 3 in the polymer film wrapping or bag 4. In this embodiment the contact area between the liquid or urine 33 and the material 3 is much smaller, so that the amount of analytes, electrolytes which elute from material 3 and influence the actual measurement in the urine or are absorbed by part 3 from the urine is limited. In this embodiment the mechanical fastening and leakage prevention can be improved with a separate polymer tape 17 sealed on the backside, outer side of the device. This tape 17 covers the slit 6 from one side and partly the end of the polymer wrapping 4 of the capillary part 3 and sticks and is glued also to the material 10. This sealing 17 can also be a printed surface of polymer such as silicone or PUR, which seals off the slit 6. Also possible is the mechanical fastening and sealing principle with the printed lines 13 as shown in fig. 6 and 11.

Also visible in this side view and cross section are the printed sticky polymer lines, rims 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, the outside. Part 7 which is the unexposed part of the capillary material 3 within part 4, is the part on which the patient drops his or her urine. The urine will be absorbed here and will transported in the capillary device material 3 to the end 34, where it drops in the bag shaped film wrapping 4.

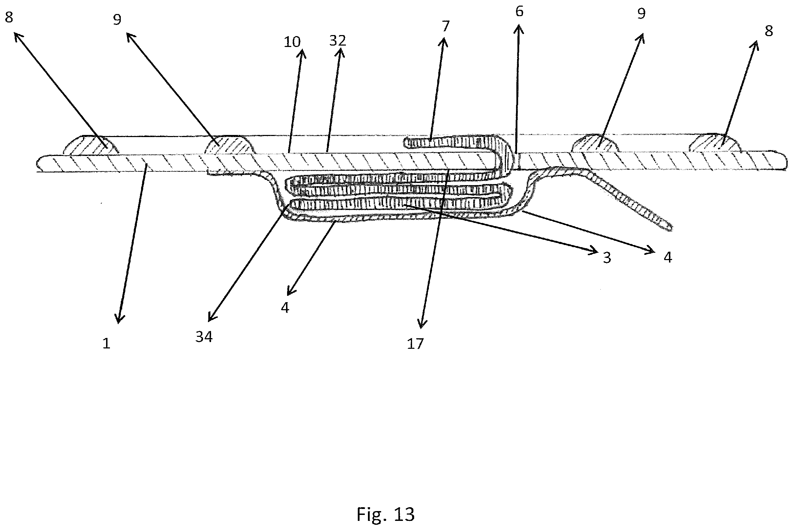

Fig. 13 shows a side view and cross section of the device with the capillary part entering the butterfly from the outer, non skin side with a tape sealing at the outer side. It shows the capillary part 3, underneath the device 1, partly folded and covered in a polymer, cup or bag shaped film 4, forming a sort of cavity, and entering the butterfly from the cup shaped part 4, via slit 6 to the inside part 10 which can be covered with a hydrophobic silicone film 32 in all embodiments. The body liquid or urine 33 is absorbed at part 7, and flows into and enters part 3 further and is absorbed subsequently totally in part 3 till it reaches the end section part 34 and the saturation is maximal, depending on the produced volume of urine. In this embodiment the mechanical fastening between capillary part 3 and part 1 is not absolutely necessary, while the polymer film cup 4 keeps it at it's place and inside the cup shape. If necessary also in this embodiment the mechanical fastening solutions of previous described embodiments can be applied. This can be a tape 17 that covers the slit 6 from one side and partly the capillary part 3 and sticks and is glued, sealed also to the material 10. This sealing 17 can also be a printed surface of polymer such as silicone or PUR, which seals off the slit 6. Also possible is the mechanical fastening and sealing principle with the printed lines 13 as shown in fig. 6 and 11. Also visible in this side view and cross section are the printed polymer lines, rims 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, the outside. Part 7 which is the unexposed part of the capillary material 3 within part 4, is the part on which the patient drops his or her urine. The urine will be absorbed here and will transported in the capillary device material 3 to the end 34, when it's fully satirized. Another embodiment of the embodiment envisaged in fig. 13 is that the user drips his or her urine on part 7, which is in this case limited in length and size and comparable to part 7 and 3 in figure 12. Due to the capillary effect the urine will be absorbed in part 3 till this is fully satirized and then it will drip into the cavity of polymer film 4, which act then as an urine collection cavity.

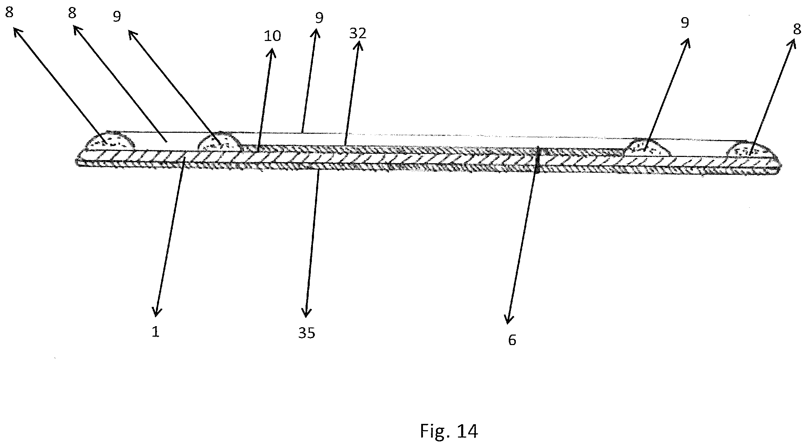

Fig. 14 shows a side view and cross section of the device without the capillary part 4 Also visible in this side view and cross section are the printed polymer lines 8 and 9 which enable fastening of the device to the skin of patient and form a double barrier to prevent entering of feces parts, which would contaminate the urine sample to be collected. Part 10 is the base material of the device which can be coated with a watertight coating, printed layer 32 of polymer, silicone, PUR or other material on the inside of part, area, section 10 and if necessary the outside, the outer and non skin side of the device (35). Part 6 is the slit which can be opened to mount the capillary part 3 with silicone wrapping (not visible in the figure).

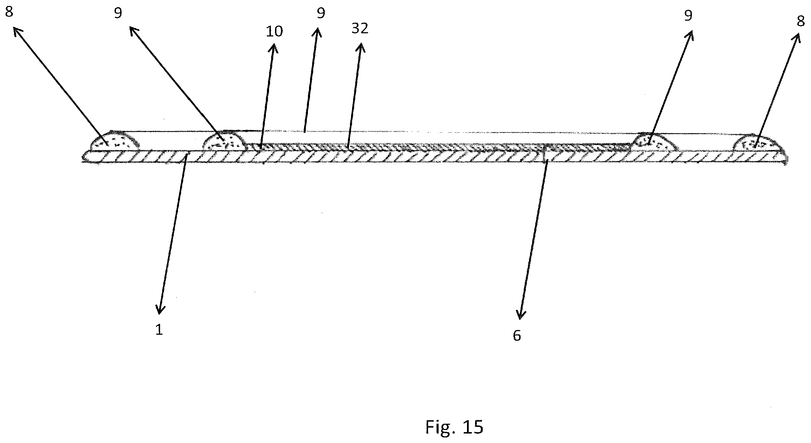

Fig. 15 shows a side view and cross section of the device without the capillary part 4 Also visible in this side view and cross section are the printed polymer lines 8 and 9 which enable fastening of the device to the skin of patient and form a double barrier to prevent entering of feces parts in the inner area 10, which would contaminate the urine sample to be collected. Part 10 is the inner area of the base material of the device which can be coated with a watertight coating, printed layer 32 of polymer, silicone, PUR or any other hydrophobic and or watertight material on the inside of this part, area 10. Part 6 is the slit which can be opened to mount here the capillary part 3 with silicone wrapping (not visible in the figure).

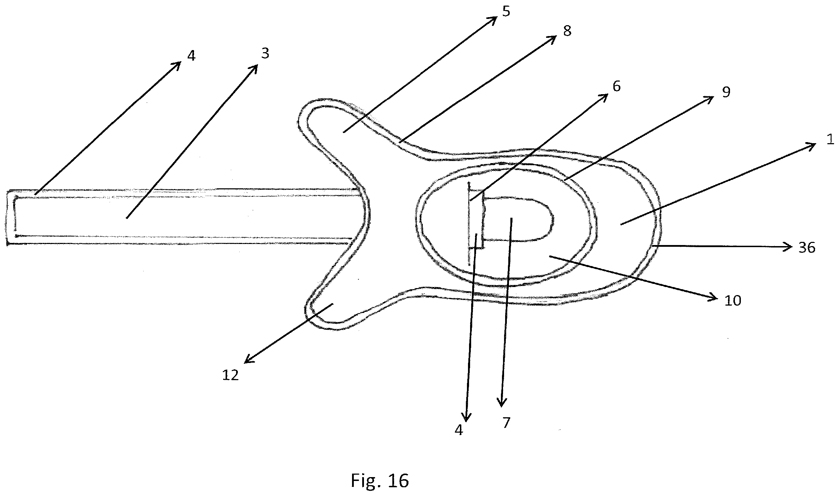

Fig 16 shows a top view of a possible embodiment of the device for female patients with the capillary part 3 parallel with the actual main part of the device 1. This main part 1 is bendable, drapeable and flexible and can adapt itself to the anatomy of the patient and follow the body contours and has therefore has a curving, large radius, oval or ellipse shape 36 at one end. Also part 3 is flexible, bendable and can put on the leg of the patient, with a part of it visible outside the diaper that the patient is wearing. Part 1 has two end sections, parts 5 and 12, which are smaller in dimension, rectangular, curved or otherwise shaped and integrally connected to the rest of part 1. These ends can facilitate a better fitting to the body contour and enable fastening of the device to it. The non existing material of part 1 between part 5 and part 12 can be used to position the device just outside the anus of patient or can be used for fastening above the genital area. Part 1 will therefore not be glued on the anus but glued around the anus and upwardly in the direction of the belly. On the other side part 1 has a curved shape, round, elliptic, oval or an kind of shape, which also can easily adapt itself to the contours of the skin and. Part 1 has at its circumference a sticky, raised line of polymer 8 which acts as an adhesion line to mount and stick the device to the skin of the patient and act as a first barrier against feces,

The capillary part 3 is connected to part 1 via a slit 6 and enters the device part 1 from the outer, non skin side. The capillary part 3 is covered in a polymer film sleeve or wrapping 4, which protects the urine or body fluids from dehydration and contamination before it is actually processed in the laboratory for diagnostic measurements. The capillary structure has a part 7, which is not covered with a polymer film, but which is protruding on the inner side of the device and is capable of absorbing the urine that the patient produces. This urine will be transported in the capillary structure of 3 upwardly.

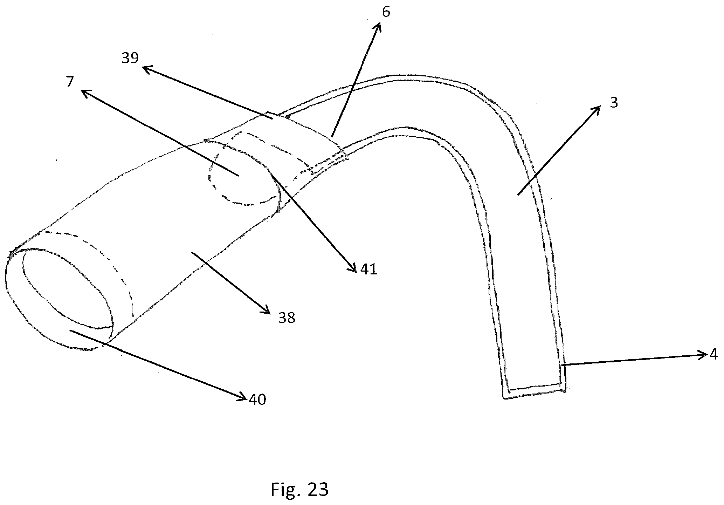

The angle between part 3 and part can be e.g. 0, 15, 30, 45 degrees or any kind of preferred angle. When the part 3 is under an angle, it can be partly underneath extension end, part 5 or extension end, part 12, so that it will be on the left or right leg of the patient, depending on the nursing practice. The main part 1 has an inner area 10, which is limited by a sticky, raised line, rim of polymer 9. This raised line, with any kind of height, can be extruded, 2D printed, or silk screen print or applied in any other matter and forms a barrier against feces and will keep the urine that the patient produces within the area 10. This area 10 is coated with an inert polymer, which preferably is hydrophobic, but can also be hydrophilic. All the urine which the patient produces will drop on this area 10 and directly on the capillary end part 7, Also the urine which isn't dropped directly on part 7, will be absorbed by this capillary part 7. The shape as shown in fig. 23 with the two ends 5 and 12 and a more larger curved part 36 at the other side, which can easily be adapted and fitted to the skin contours and anatomy directly above the genital area of the patient, can also be different and have other base shapes and outlines, such as a completely oval or ellipse shape, rectangular shape with or without radii in the corners, tri-angular shape with or without radii in the edges, a hexagonal, a octagonal, an irregular shape etc. Essential is that the device consist of the two main parts: part 1 which is connected to skin via the polymer lines, rims 8 and 9 and part 3 wrapped in polymer part 4, which is able to absorb urine or body fluids via the exposed and not wrapped part 7 which is directly in the vicinity of the urethra in case of a female patient and the tip of the penis in case of a male patient.

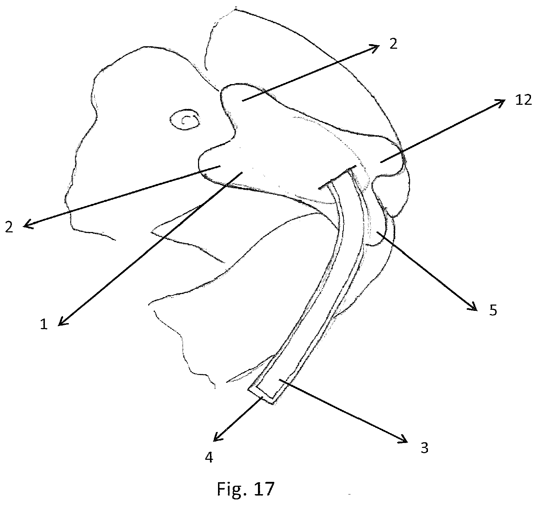

Fig. 17 shows a 3D view of the device mounted on a female baby. The device has a capillary part 3 covered in polymer wrapping part 4 which is positioned on the leg of the female baby, which is drawn in the figure underneath this part 3. Part 5 and 12 are positioned around the anus and will not cover this anatomy part The device can also be put 180 degrees turned on the patient if necessary and then ends, parts 2 will be around the anus, and part 3 will be directed upwardly and will be not positioned on the leg but on the belly of the patient

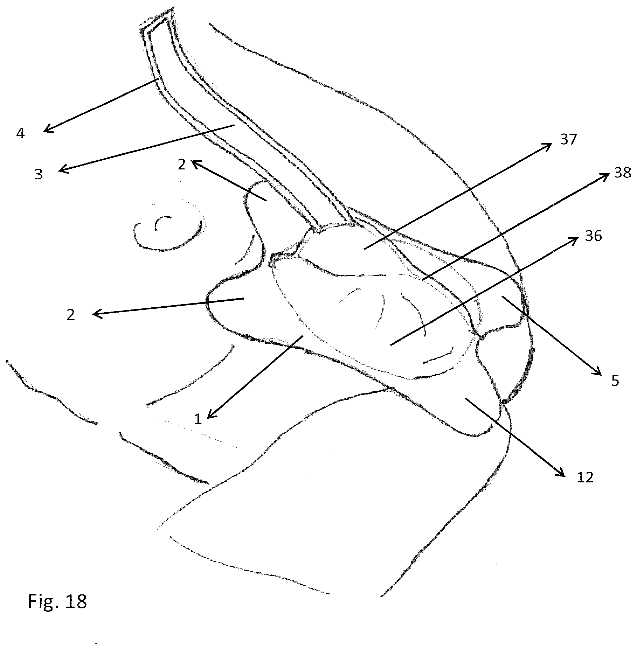

Fig. 18 shows a 3D view of an embodiment the device intended for and mounted on a male baby. The device has a capillary part 3 covered in polymer wrapping part 4 which is positioned on the belly or leg of the male baby. Parts 5 and 12 are positioned around the anus and will not cover this anatomy part The device can also be put 180 degrees turned on the patient's skin and genital area if necessary and then the ends, parts 2 will be around the anus, and part 3 will be directed upwardly and will be not positioned on the leg but on the belly of the patient The male version has a cavity shaped part 36 which covers the base part of the penis of the young male patient and his testicles. The top of this part 36 ends in a smaller part 37 which can accommodate the tip of the penis of the patient The cavity shape can consist of two parts which are sealed together at the seal rim 38. The cavity shapes 36 and 37 can also be shaped integrally from base part 1 with thermoforming of textile or polymer material of part 1 in a heat/press system, and will in this special embodiment not have a sealing rim 38, Mounted in the smaller cavity shaped end part 37 is the capillary part 3, which is covered in a polymer film wrapping 4. This male version with an integrated cavity, creating a hollow shape can also be applied to female patients.

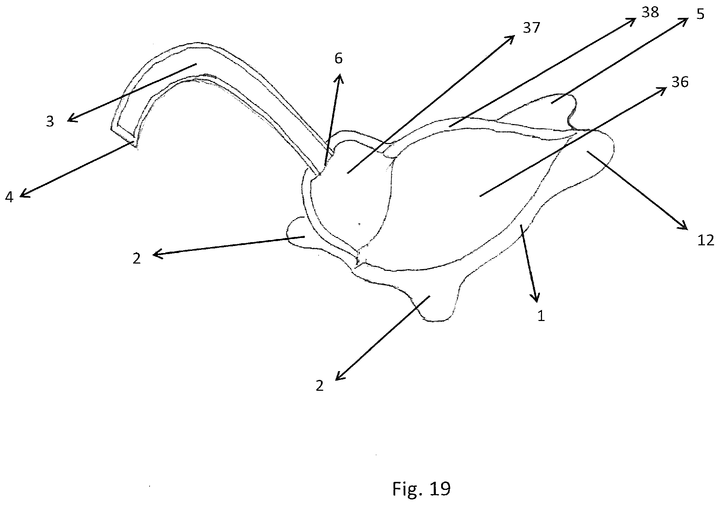

Fig 19 shows a 3D view of another possible embodiment of a male device. The device has a capillary part 3 covered in polymer wrapping part 4 which is positioned on the belly or leg of the male baby. Parts 5 and 12 are extended end and can be positioned around the anus and will not cover this anatomy part, the small part 2 are draped and sticked to skin of the lower part of the belly. The male version has a cavity shaped part 36 which covers the base part of the penis of the young male patient and his testicles. The top of this part 36 ends in a smaller part 37 which can accommodate the tip of the penis of the patient. The cavity shape can consist of two parts which are sealed together at the seal rim 38. Mounted in the smaller cavity shaped end part 37, via the slit 6 is the capillary part 3, which is covered in a polymer film wrapping 4. Also in this embodiment the slit 6 can be sealed or hermetically closed with a sticky polymer inner lining. This male version with cavity can also be applied to female patients.

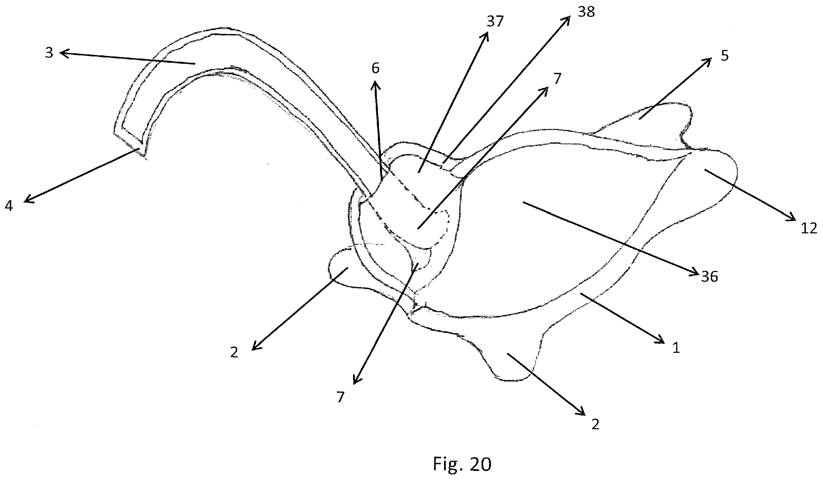

Fig. 20 shows a 3D view of a possible embodiment of a male device. shows a 3D view of another possible embodiment of a male device. The device has a capillary part 3 covered in polymer wrapping part 4 which is positioned on the belly or leg of the male baby. Parts 5 and 12 are extended end and can be positioned around the anus and will not cover this anatomy part, the small part 2 are draped and sticked to skin of the lower part of the belly. The male version has a cavity shaped part 36 which covers the base part of the penis of the young male patient and his testicles. The top of this part 36 ends in a smaller part 37 which can accommodate the tip of the penis of the patient The cavity shape can consist of two parts which are sealed together at the seal rim 38. Mounted in the smaller cavity shaped end part 37, via the slit 6 is the capillary part 3, which is covered in a polymer film wrapping 4. Part 3 has one, two or more ends 7, which are not covered by the polymer film wrapping 4, and which are drawn with dotted lines in the drawing. These ends are close to or in contact with the urethra of the male penis. They are able to absorb the urine fast and transport it further in the capillary part 3. Also in this embodiment the slit 6 can be sealed or hermetically closed with a sticky polymer inner lining.

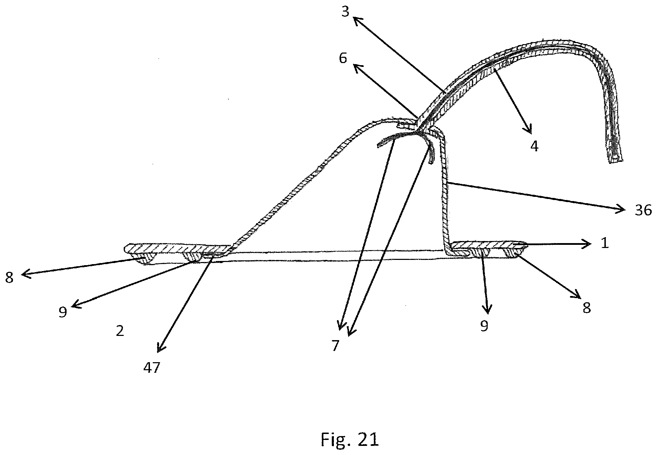

Fig. 21 shows a cross section of a possible embodiment of the device. The device has a capillary part 3 covered in polymer wrapping part 4 which is positioned on the belly or leg of the male baby or female baby. This male and female version has a cavity shaped part 36 which covers the base part of the penis of the young male patient and his testicles or the vulva and urethra of the female patient. The cavity shape can consist of one part or two parts which are sealed together. Cavity part 36 can be sealed or glued via the rims 47 to the bottom side of part 1. Or cavity part 36 is integrally thermoformed from a sheet of base material. Mounted in cavity shaped end part 36, via the slit 6 is the capillary part 3, which is covered in a polymer film wrapping 4. Part 3 has one, two or more ends 7, which are not covered by the polymer film wrapping 4, and which are drawn with dotted lines in the drawing. These ends are close to or in contact with the urethra or the male penis. They are able to absorb the urine fast and transport it further in the capillary part 3. Also in this embodiment the slit 6 can be sealed or hermetically closed with a sticky polymer inner lining. The part 1 is glued to the skin and lower belly around the penis and testicles with the sealing rims 8 and 9.

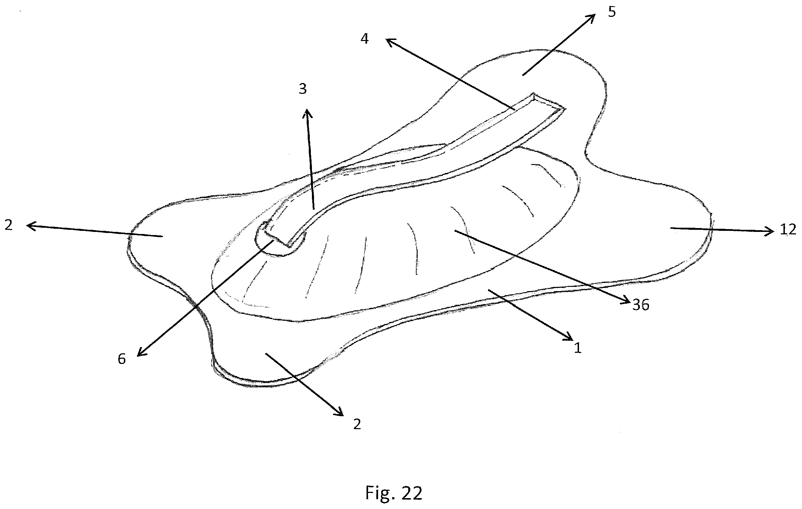

Fig. 22 shows a 3D view of a possible embodiment of a male device

The device has a capillary part 3 covered in polymer wrapping part 4 which is positioned on the belly or leg of the male baby. Parts 5 and 12 are extended end and can be positioned around the anus and will not cover this anatomy part, the small part 2 are draped and sticked to skin of the lower part of the belly. The male version has a cavity shaped part 36 which covers the base part of the penis of the young male patient and his testicles. This part 36 can be formed by pressing with heat a preformed stamp of mold part onto the base material of part 1, which then will be moulded into a cup, cavity like shape. This part 36 can have in a specific embodiment a smaller cup shape which can accommodate the tip of the penis of the patient Mounted in the cavity shaped end part 36, via the slit 6 is the capillary part 3, which is covered in a polymer film wrapping 4. Also in this embodiment the slit 6 can be sealed or hermetically closed with a sticky polymer inner lining. This male version with cavity can also be applied to female patients.

Fig. 23 shows a 3D view of a possible embodiment of a device. The device has a capillary part 3 covered in polymer wrapping part 4 which is positioned on the belly or leg of the male baby. This male version has a cylindrical shaped part 38 which covers the shaft and tip of the penis of the young male patient. The part 38 can be made with circular knitting of textile fibers in one piece. After that it can be coated at the inside and possible outside with a polymer coating. Part 38 can also be made of any kind of flat sheet material, polymer film, textile or combinations thereof, which is sealed over the length to create a cylinder. This part 38 can have on the inside a sticky or non sticky polymer coating 40 which can prevent leakage of urine at this point. Preferably this is a sticky silicone adhesive which sticks to the skin and is watertight. This part 38 has a flatter end and partly cylindrical part 39 after the line 41 which is pressed with forced and het in the cylinder material, which contains on the inside the non exposed part 7 of the capillary material 3. Mounted in part 39 via the slit 6 is the capillary part 3, which is covered in a polymer film wrapping 4. Also in this embodiment the slit 6 can be sealed or hermetically closed with a sticky polymer inner lining.

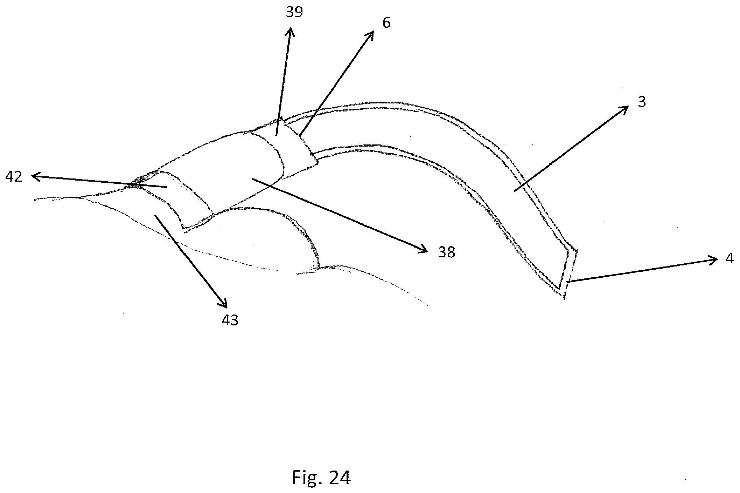

Fig. 24 shows a 3D view of a possible embodiment of a possible embodiment of the device and show as mounted on the penis of the young male patient. The device has a capillary part 3 covered in polymer wrapping part 4 which can be positioned on the belly or leg of the male baby. This male version has a cylindrical shaped part 38 which covers the shaft and tip of the penis of the young male patient. This part 38 can have on the outside a reinforcement rim 40 near the beginning of the shaft of the penis 43, which is elastic and has sufficient strength to prevent sliding of the part from the shaft of the penis and prevent leakage via a close skin contact. On the inside a sticky or non sticky polymer material can be applied which can contribute also to prevention of leakage of urine at this point Preferably this is a sticky silicone adhesive which sticks to the skin and is watertight This part 38 has a flatter end and partly cylindrical part 39 which contains on the inside the non exposed part 7 of the capillary material 3. Mounted in part 39 via the slit 6 is the capillary part 3, which is covered in a polymer film wrapping 4. Also in this embodiment the slit 6 can be sealed or hermetically closed with a sticky polymer inner lining.

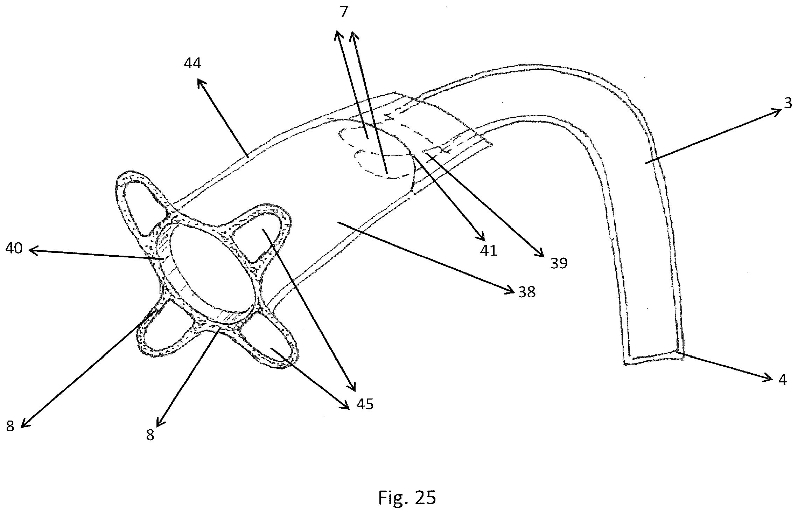

Fig. 25 shows a 3D view of a possible embodiment of a male device. The device has a capillary part 3 covered in polymer wrapping part 4 which is positioned on the belly or leg of the male baby. This male version has a cylindrical shaped part 38 which covers the shaft and tip of the penis of the young male patient. The part 38 can be made with circular knitting of textile fibers in one piece. After that it can be coated at the inside and possible outside with a polymer coating. Part 38 can also be made of any kind of flat sheet material, polymer film, textile or combinations thereof, which is sealed over the length to create a cylinder. This part 38 can have on the inside a sticky or non sticky polymer coating 40 which can prevent leakage of urine at this point and prevent shifting and sliding of the shaft due to a higher friction. Preferably this is a sticky silicone adhesive which sticks to the skin and is watertight. Part 38 can also be made of one or two sheets of polymer or textile material which are sealed together at sealing line(s) 44. This part 38 has a flatter end and partly cylindrical part 39 after the line 41 which is pressed with forced and het in the cylinder material, which contains on the inside the non exposed parts 7 of the capillary material 3. Mounted in part 39 via the slit 6 is the capillary part 3, which is covered in a polymer film wrapping 4. Also in this embodiment the slit 6 can be sealed or hermetically closed with a sticky polymer inner lining. The cylinder part 38 has two or more ends 45, which have printed adhesive lines 8 and 9 and can be sticked to skin to ensure proper fastening and prevent outward leakage of urine and inward leakage of fecal parts.

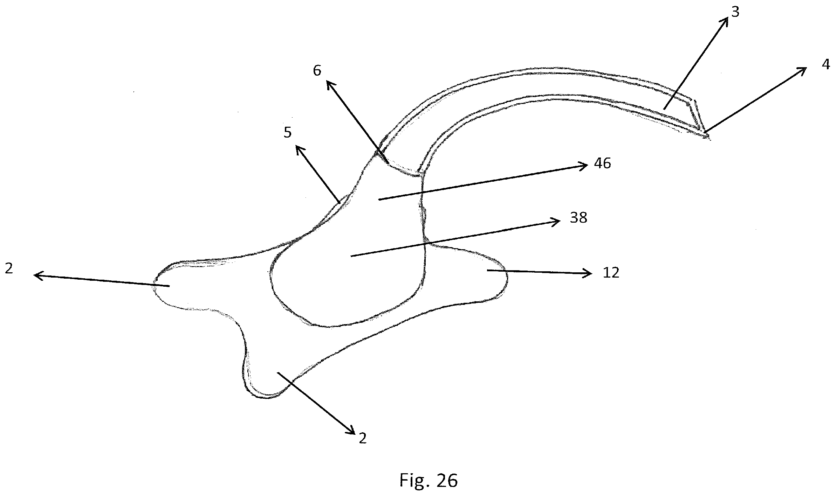

Fig. 26 shows a 3D view of a possible embodiment of a male device. The device has a capillary part 3 covered in a polymer film and wrapping part 4 which can be positioned on the belly or leg of the baby. Parts 5 and 12 are extended ends and can be positioned around the anus and will not cover this. This male version has a partly dome and partly cylindrical shaped part 38 which covers the shaft and tip of the penis of the young male patient. This part 38 can have on the outside a reinforcement rim 40 near the beginning of the shaft of the penis 43, which is elastic and has sufficient strength to prevent sliding of the part from the shaft of the penis and prevent leakage via a close skin contact. On the inside a sticky or non sticky polymer material can be applied which can contribute also to prevention of leakage of urine at this point Preferably this is a sticky silicone adhesive which sticks to the skin and is watertight. This dome shaped part 38 has a partly cylindrical part and flattened part 46 which contains on the inside the non exposed part 7 of the capillary material 3. Mounted in part 46 via the slit 6 is the capillary part 3, which is covered in a polymer film wrapping 4. Also in this embodiment the slit 6 can be sealed or hermetically closed with a sticky polymer inner lining.

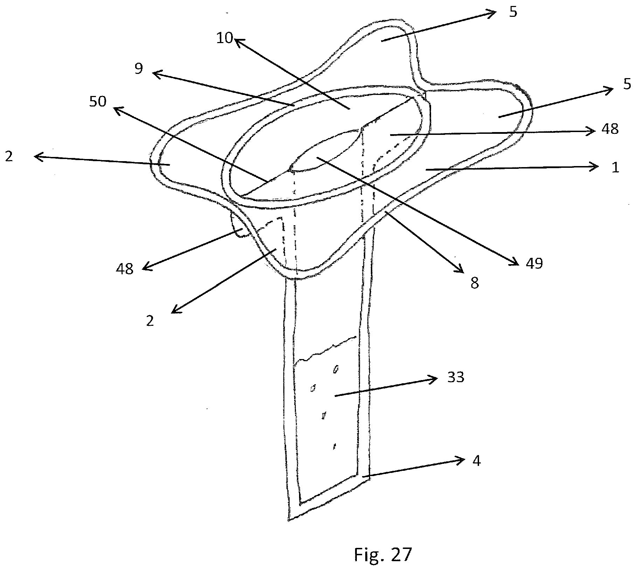

Fig. 27 shows a 3-dimensional drawing of the device with a central opening connection to a small urine collection compartment. The liquid or urine 33 is collected within the area 10, and enters part 4 at the central opening 49. further and the drips subsequently from the end 34 of part 3 in the polymer film wrapping or bag 4.

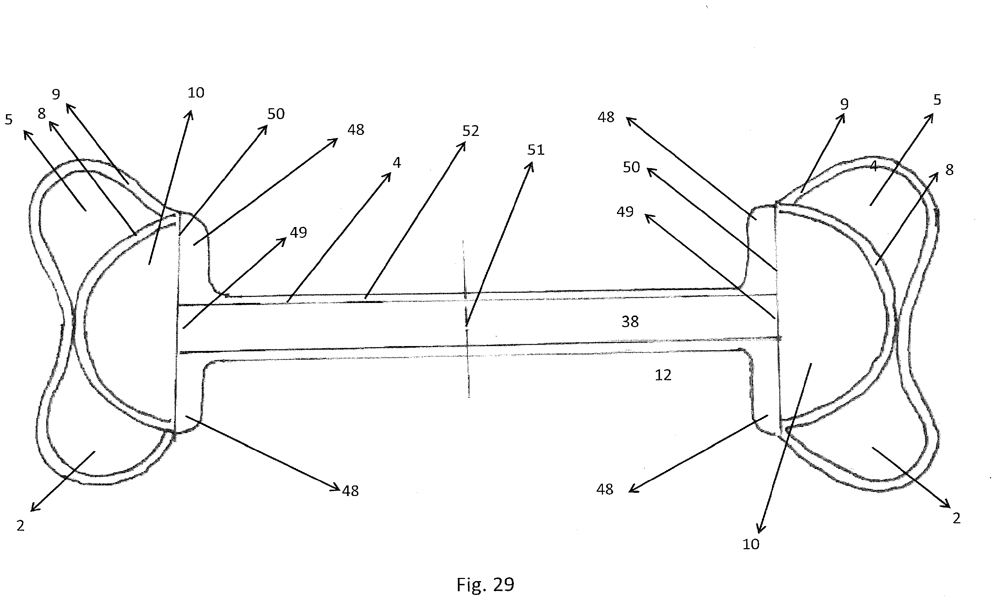

Also visible in this 3D view are the printed polymer lines 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, the outside. The base part 1 has rounded ends 2 and 5 to enable proper fastening at the 3D-shape of the skin. The device in this embodiment is made in flat condition (see fig. 29) is joined at the sealing or flange area 48, which is on the non skin side. At the skin side only the upper part of this sealing rim visible in the shape of sealing line 50

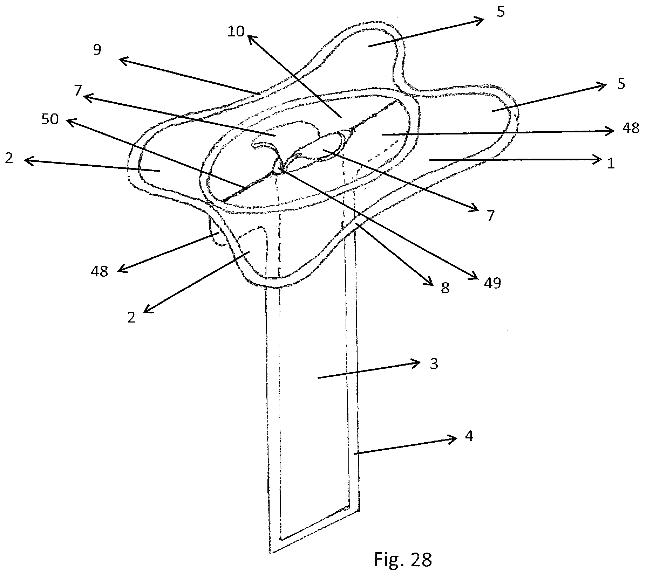

Fig. 28 shows a 3-dimensional drawing of the device with a central opening connection to a small urine collection compartment. The liquid or urine 33 is collected on the rounded ends 7 of the capillary material 3, upwardly sticking out within the area 10 from opening 49, and is contained in the polymer film forming collection compartment part 4.

Also visible in this 3D view are the printed polymer lines 8 and 9 which enable fastening of the device to the skin of patient and form a double barrier to prevent entering of feces parts, which would contaminate the urine sample to be collected. Part 10 is the base material of the device which can be coated with a watertight polymer on the inside and if necessary, the outside. The base part 1 has rounded ends 2 and 5 to enable proper fastening at the 3D-shape of the skin. The device in this embodiment is made in flat condition (see fig. 29) is joined at the sealing or flange area 48, which is on the non skin side.

Fig. 29 shows a 3-dimensional drawing of the device in plano, flat, unfolded pre final production stage or condition before the actual sealing takes place. The device is fold along the central center line 51 and the sealed at the flanges 48 and the rims 52 of the polymer film material which will create the compartment 4.

Also visible in this 3D view are the areas where sticky polymer lines 8 and 9 will be printed. The base part 1 has rounded ends 2 and 5 to enable proper fastening at the 3D-shape of the skin. The opening 29 at the area where part 4 connects to part 1 can be enlarged by mechanical deformation in an ellipse shape to enable easy dropping of the urine 33 in compartment 4 in case of the embodiment of fig. 27, without the use of capillary material 3.

Fig 30 shows a 2D view of a possible embodiment of a female device made in one integral piece. The base or main part 1 is now made in one piece of a textile material together with the integrated capillary part 3 consisting of the same base material. It is cut in one piece from a suitable textile material or capillary active material, which can act as drapeable part and as capillary part the same time. It is coated on both sides with e.g. a printed, e.g. silk screen printed silicone layer or comparable watertight polymer layer, excluded the section of part 3 which is not coated and therefore capable of absorbing liquid such as urine. This printed coating is on the inner side coated on areas 53 and 10 and underneath the later extruded adhesive printed lines 8 and 9. On the outer, non skin side the textile is coated on the whole surface of the base part with integrated capillary part. Part 3 is subsequently covered with a silicone film 4, which is glued along the lines 52. Part or section 3 has via the silicone coatings and films, liquid barriers on 3 directions to prevent leakage and wicking of liquid such as urine from section 1 to other areas of the device. At the back side it is a printed silicone layer (not visible in drawing), at the top and inner part of the textile material along the edges of part 3 also a printed or coated layer (51,52). The top surface is covered with a separate silicone film 4. As alternative to inert silicone other polymers can be applied or other treatment which makes the textile material complete hydrophobic on the surroundings of the part which has to be hydrophilic and fulfil the capillary function. As a final production step the adhesive printed lines 8 and 9 are printed on the inner side, also covering part of the polymer film 4 cover of part 3. This main part 1 is bendable, drapeable and flexible and can adapt itself to the anatomy of the patient and follow the body contours. Also the integrated part 3 is flexible, bendable and can be put on the leg of the patient, with a part of it visible outside the diaper that the patient is wearing. Part 1 has one or more ends which are smaller in dimension, rectangular, curved or otherwise shaped and integrally connected to the rest of part 1. These ends can facilitate a better fitting to the body contour and enable fastening of the device to it The capillary part 3 is covered in a polymer film sleeve or wrapping 4, which protects the urine or body fluid from dehydration and contamination before it is actually processed in the laboratory for diagnostic measurements. The capillary structure 3 has again an end part 7, which is not covered with a polymer film 4, but which is exposed on the inner side of the device and is capable of absorbing the urine that the patient produces initially at fisrt. This urine will be then transported in the capillary structure of part 3 upwardly.

The areas, parts 10, 53 are coated, printed with an inert polymer, which preferably is hydrophobic, or are treated with a method and or finishing system to make them hydrophobic, but can also be hydrophilic or combinations of both. An example or possible embodiment is the use of a non sticky silicone finish or coating applied by any kind of process. Part 10 is an area which is watertight and can't absorb urine. All the urine which the patient produces will drop on this area 10 and directly on the capillary end part 7, Also the urine which isn't dropped directly on part 7, will be absorbed by this capillary part 7, due to the inability of area 10 to absorb urine. Part 1 can be one integral part with area 10. It can consist of woven, knitted or non woven synthetic or natural textile material. Parts 10, and 53 can be made watertight and or hydrophobic via a polymer coating, such as a silicone, PUR, Polyacetate, ink coating, or via a plasma treatment. The areas or parts 10, 53 can also gave a separate water tight part in the shape of a in contour shaped cut silicone film which is glued, sealed or mechanically fasted to part, inner side and or outer side. Also part 1 can be made of an inert polymer, on which is glued a capillary part 3, such as capillary textile or foam material, which is also protected with polymer film 4. Lines 8 and 9 with the sticky polymer are printed on this cover 4, to enable adhesion to the skin and inward and outward leakage via the skin.

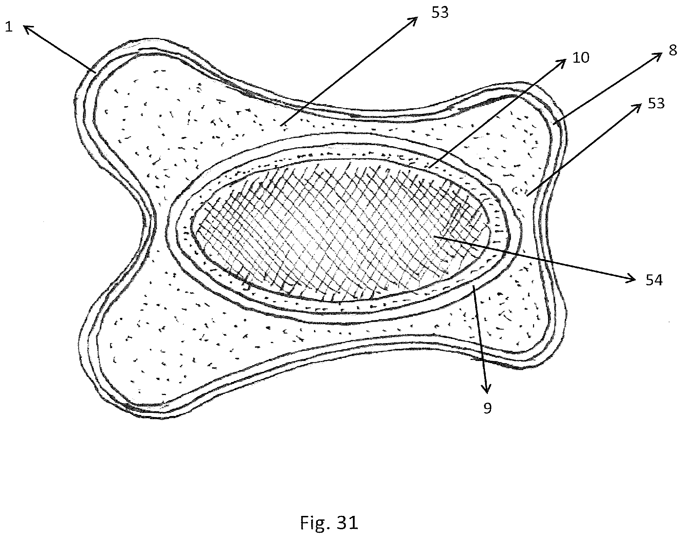

Fig 31 shows a 2D view of a possible embodiment of a device for collection of perspiration with a single, large collection area. The base or main part 1 is made in one piece of a textile material together with the integrated capillary area 54 consisting of the same base material. It is cut in one piece from a suitable textile material or capillary active material, which can act as drapeable part and as capillary part the same time. It is coated on at least the whole are of the back side, being the non skin side with e.g. a printed, e.g. silk screen printed silicone layer 10 or comparable watertight polymer layer. This watertight layer 10 is also printed on the skin side, being the collection side of the perspiration vapours and droplets around the rim of the collection area 54 and can be printed on areas 53 also on the inside (skin-side) as well as the outside. This collection area is the surrounded at the rims and at the bottom with an impermeable polymer coating 10. This material 10 is fully penetrating the thickness of the textile material of the device till it creates a contact with the coating 10 printed on the bottom side. By this a vertical liquid barrier is created within the material. At the rim a sticky, preferably compressible polymer line 9 is printed on the rim coating 10. This polymer line 9 can be attached to the skin, creating by this a closed perspiration collection area. The collection area 54 can absorb perspiration droplets and perspiration vapours will condensate on and in the inner capillary structure of area 54. This collection area 54 can hermetically be sealed with these printed polymer lines 9 to the skin, preventing by this the permeation and escape of the perspiration vapours and droplets to the environment and retaining them within the collection area 54. The collection area 54 can also fully be coated with the hydrophobic and watertight polymer layer 10, in which case the perspiration will accumulate as droplets on this surface till the volume of the collection area is filled till the height of the polymer rim 9. Part 54 has via the silicone coatings and films, liquid barriers on 3 directions (X, Y, Z) to prevent leakage and wicking of liquid such as perspiration from section 54 to other areas of the device.

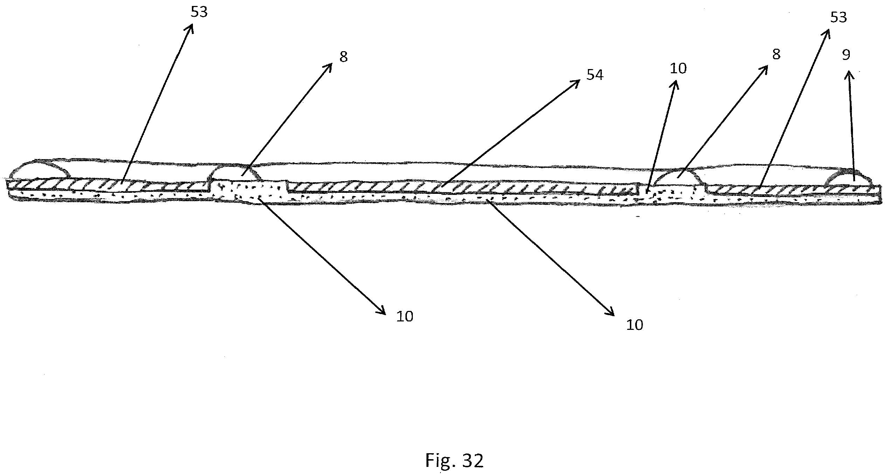

Fig 32 shows a 2D cross section of a possible embodiment of a device for collection of perspiration. The base or main part 1 is made in one piece of a textile material or other capillary material including the integrated capillary area 54 consisting of the same base material. It is cut in one piece from a suitable textile material or capillary active material, which can act as drapeable part and as capillary part the same time. It is coated on at least the whole are of the back side, being the non skin side with e.g. a printed, e.g. silk screen printed silicone layer 10 or comparable watertight polymer layer. This watertight layer 10 is also printed on the skin side, being the collection side of the perspiration vapours and droplets around, at the rim of the collection area 54 and possibly in areas 53 at the outer side and if necessary skin side. This collection area is the surrounded at the rims and at the bottom with an impermeable polymer coating 10, which is also the printing area sticky rim, line, 9 realized preferably with a compressible sticky polymer, which is printed on the rim coating 10. This polymer line, rim 9 can be attached to the skin, creating by this a closed perspiration collection area. The collection area 54 can absorb perspiration droplets and vapours will condensate at an in the inner capillary structure of area 54. The collection area 54 can also fully be coated with the hydrophobic and watertight polymer layer 10, in which case the perspiration will accumulate as droplets on the surface till it filled till the height of the polymer rim 9. Part 54 has via the silicone coatings and films, liquid barriers on 3 directions to prevent leakage and wicking of liquid such as perspiration from section 54 to other areas of the device.

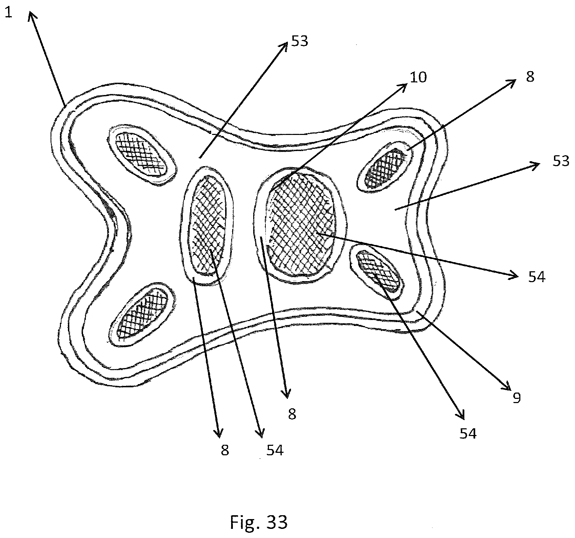

Fig 33 shows a 2D view of a possible embodiment of a device for collection of perspiration with multiple collection areas, to be able to collect perspiration at several areas simultaneously and with a higher chance of retaining the perspiration due to smaller collection areas which are hermetically sealed to the skin of the patient. The base or main part 1 is made again in one piece of a textile material together with the integrated capillary area 54 consisting of the same base material. It is cut in one piece from a suitable textile material or capillary active material, which can act as drapeable part and as capillary part the same time. It is coated on at least the whole are of the back side, being the non skin side with e.g. a printed, e.g. silk screen printed silicone layer 10 or comparable watertight polymer layer. This watertight layer 10 is also printed on the skin side, being the collection side of the perspiration vapours and droplets around, at the rim of the multiple collection areas 54. These collection areas are surrounded at the rims and at the bottom with an impermeable polymer coating 10 and are smaller than in the embodiment of fig, 31 and 32. At the rim, edge of the collection area a sticky, realised preferably compressible polymer line, rim 9 is printed on the rim coating 10. This polymer line 9 can be attached to the skin, creating by this a closed respiration collection area. The smaller size and the reduced amount of air within the closed collection areas will slow down possible evaporation, degradation and or permeation of the perspiration. The collection areas 54 can absorb perspiration droplets and perspiration vapours will condensate at and in the inner capillary structure of area 54. The collection areas 54 can also fully be coated with the hydrophobic and watertight polymer layer 10, in which case the perspiration will accumulate as droplets on the surface till it filled till the height of the polymer rim 9. Part 54 has via the silicone coatings and films, liquid barriers on 3 directions to prevent leakage and wicking of liquid such as perspiration from section 54 to other areas of the device.

The possible embodiments of the invention are not limited to the described and visualized embodiments in the above sections and detailed descriptions of figures, but encompass modifications and combinations of features disclosed in the application. The scope of the invention is defined by the appended claims solely.

## Claims

1. A system for collecting samples of urine or body fluids exuding from the skin of a user, comprising a base part (1) of a pliable material adapted to be draped on the skin of a user closely following the contours of a predetermined body part of the user and having an inner side facing the skin of the user, and a urine or fluid collecting part (3) provided on the inner side of the base part and comprising a capillary structure (3) which is at least partly enclosed by a polymer film wrapping or bag (4) adapted to receive and contain urine or fluids excreted by a user, wherein the base part, on its inner side, comprises at least an area (10) defined by at least one second barrier line or rim (9) encircling at least a part of the urine or fluid collecting part (3) exposed to a user, and at least a first barrier line or rim (8) at its circumference encircling at least one second barrier line or rim, and wherein
the barrier lines or rims are adapted to sealingly engage the skin of a user, thereby preventing intrusion of any contaminations into the area (10) surrounding the urine or fluid collecting part and leakage of urine or body fluids therefrom.

2. The system of claim 1, wherein the barrier lines or rims (9,8) have the shape of one of a raised area, a film, a printed area or line or any structure adapted to effect hermetically sealing against the skin of a user.

3. The system of claim 1 or 2, wherein the barrier lines or rims (9,8) have adhesive characteristics on a surface providing efficient adhesion to the skin of a user and an easy peel-off characteristic.

4. The system of any of the preceding claims, wherein the barrier lines (9,8) comprise flexible and/or cushioning material able to closely follow the skin contours of an user.

5. The system of any of the preceding claims, wherein on the surface of the area of the inner side of the base part (1) between the first and second barrier lines or rims (9,8) protruding dots and or rims are provided having the same height as the barrier lines or rims.

6. The system of any of the preceding claims, wherein the area (10) of the base part (1) is coated with an inert material unable to influence essential characteristics of urine or body fluids received in this area.

7. The system of any of the preceding claims, wherein the base part (1) is made of a textile or textile based pliable material.

8. The system of claim 7, wherein the textile or textile based material is coated on one or both of its sides with a waterproof or water repellent inert material.

9. The system of any of the preceding claims, wherein the base part (1) is made of a waterproof or water repellent material.

10. The system of any of the preceding claims, wherein the base part (1) is shaped to surround and cover the genital area of a user, leaving the user's anus area uncovered.

11. The system of any of the preceding claims, wherein the base part (1) has rounded edges or corners.

12. The system of any of the preceding claims, wherein the base part (1) has at least one or two laterally protruding parts (2,12).

13. The system of any of the preceding claims, wherein the urine or body fluid collecting part (3) is extending from a barrier-defined waterproof or water repellent area (10) on the inner side of the base part (1) to an outer side of the base part and that it comprises at least one inner end part (7) located within the area (10) and capable of receiving urine or body fluids, and an outer part protruding from the base part (1).

14. The system of claim 13, wherein the outer part of the urine or body fluid collecting part (3) is covered by a protective cover made of an opaque or a transparent material (4).

15. The system of claim 14, wherein the protective cover (4) is detachably connected to the base part (1).

16. The system of claim 13, wherein the outer part of the urine or body fluid collecting part (3) is at least partly extending underneath the inner uncovered end part (7) of the latter either parallel to a center line of the base part (1) or enclosing an angle therewith.

17. The system of claim 13, wherein the urine or body fluid collecting part (3) is extending through an opening (6) in the base part (1) and that sealing means (14, 17) are provided adjacent the opening on the inner side and/or outer side of the base part (1).

18. The system of any of the preceding claims, wherein the capillary structure of the urine or body fluid collecting part (3) comprises a fabric in the form of one of a single sheet, a double or multiple folded sheet, or a material having similar characteristics with one or more end parts (7).

19. The system of any of the preceding claims, wherein the capillary structure of the urine or body fluid collecting part (3) comprises one of synthetic yarns, multifilament yarns, fibers, and porous material having a cellular structure.

## Patentansprüche

1. System zum Sammeln von Proben von Urin oder von aus der Haut eines Benutzers austretenden Körperflüssigkeiten, aufweisend,
ein Basisteil (1) aus einem flexiblen Material, das dazu eingerichtet ist, auf die Haut eines Benutzers aufgelegt zu werden und sich dabei eng an die Konturen eines vorbestimmten Körperbereichs des Benutzers anzuschmiegen und das eine der Haut des Benutzers zugewandte Innenseite aufweist, und
ein an der Innenseite des Basisteils vorgesehenes Urin- oder Körperflüssigkeitssammelteil (3), das eine Kapillarstruktur (3) umfasst, die zumindest teilweise von einer Umhüllung oder einem Beutel (4) aus Polymerfolie umschlossen ist, die bzw. der dazu eingerichtet ist, von einem Benutzer ausgeschiedenen Urin oder ausgeschiedene Körperflüssigkeit aufzunehmen und darin zurückzuhalten,
wobei das Basisteil an seiner Innenseite wenigstens einen Bereich (10) aufweist, der durch wenigstens eine zweite Barrierelinie oder einen zweiten Barriererand (9) begrenzt ist, die bzw. der wenigstens einen zum Benutzer hin freiliegenden Teil des Urin- oder Körperflüssigkeitssammelteils (3) umgibt und wenigstens eine erste Barrierelinie oder einen ersten Barriererand (8) an seinem Umfang aufweist, die bzw. der wenigstens eine zweite Barrierelinie oder einen zweiten Barriererand umgibt, und
wobei die Barrierelinien oder Barriereränder dazu eingerichtet sind, dichtend an der Haut eines Benutzers anzuliegen und dadurch das Eindringen von Verunreinigungen in den das Urin- oder Körperflüssigkeitssammelteil umgebenden Bereich (10) sowie ein Austreten von Urin oder Körperflüssigkeiten aus diesem Bereich zu verhindern.

2. System nach Anspruch 1, wobei die Barrierelinien oder Barriereränder (9,8) als erhabener Bereich, als Folie, als aufgedruckter Bereich oder aufgedruckte Linie oder als eine andere Struktur ausgebildet sind, die dazu eingerichtet ist, eine hermetische Abdichtung gegenüber der Haut eines Benutzers zu bewirken.

3. System nach Anspruch 1 oder 2, wobei die Barrierelinien oder Barriereränder (9,8) an einer Oberfläche Hafteigenschaften aufweisen, die eine wirksame Haftung an der Haut eines Benutzers und ein leichtes Ablösen von dieser ermöglichen.

4. System nach einem der vorhergehenden Ansprüche, wobei die Barrierelinien oder Barriereränder (9,8) ein flexibles und/oder polsterndes Material umfassen, das dazu ausgebildet ist, den Hautkonturen eines Benutzers eng zu folgen.

5. System nach einem der vorhergehenden Ansprüche, wobei auf der Oberfläche des Bereichs der Innenseite des Basisteils (1) zwischen der ersten und der zweiten Barrierelinie, bzw. dem ersten und dem zweiten Barriererand (9,8) vorspringende Noppen und/oder Ränder vorgesehen sind, die dieselbe Höhe wie die Barrierelinien oder Barriereränder aufweisen.

6. System nach einem der vorhergehenden Ansprüche, wobei der Bereich (10) des Basisteils (1) mit einem inerten Material beschichtet ist, das die wesentlichen Eigenschaften des in diesem Bereich aufgenommenen Urins oder der in diesem Bereich aufgenommenen Körperflüssigkeiten nicht beeinflussen kann.

7. System nach einem der vorhergehenden Ansprüche, wobei das Basisteil (1) aus einem textilen oder textilbasierten flexiblen Material besteht.

8. System nach Anspruch 7, wobei das textile oder textilbasierte Material auf einer oder beiden Seiten mit einem wasserundurchlässigen und wasserabweisenden inerten Material beschichtet ist.

9. System nach einem der vorhergehenden Ansprüche, wobei das Basisteil (1) aus einem wasserundurchlässigen oder wasserabweisenden Material besteht.

10. System nach einem der vorhergehenden Ansprüche, wobei das Basisteil (1) derart geformt ist, dass es den Genitalbereich eines Benutzers umgibt und abdeckt, während der Analbereich des Benutzers unbedeckt bleibt.

11. System nach einem der vorhergehenden Ansprüche, wobei das Basisteil (1) abgerundete Kanten oder Ecken aufweist.

12. System nach einem der vorhergehenden Ansprüche, wobei das Basisteil (1) wenigstens einen oder zwei seitlich vorspringende Abschnitte (2,12) aufweist.

13. System nach einem der vorhergehenden Ansprüche, wobei sich das Urin- oder Körperflüssigkeitssammelteil (3) von einem durch eine Barriere begrenzten wasserundurchlässigen oder wasserabweisenden Bereich (10) an der Innenseite des Basisteils (1) bis zu einer Außenseite des Basisteils erstreckt und wenigstens einen innerhalb des Basisteils (10) angeordneten inneren Endabschnitt (7) der zur Aufnahme von Urin- oder Körperflüssigkeiten ausgebildet ist, sowie einen aus dem Basisteil (1) hervorstehenden äußeren Abschnitt umfasst.

14. System nach Anspruch 13, wobei der äußere Abschnitt des Urin- oder Körperflüssigkeitssammelteils (3) von einer Schutzhülle (4) aus einem opaken oder transparenten Material bedeckt ist.

15. System nach Anspruch 14, wobei die Schutzhülle (4) lösbar mit dem Basisteil (1) verbunden ist.

16. System nach Anspruch 13, wobei sich der äußere Abschnitt des Urin- oder Körperflüssigkeitssammelteils (3) zumindest teilweise unterhalb des inneren, unbedeckten Endabschnitts (7) desselben erstreckt und zwar entweder parallel zu einer Mittellinie des Basisteils (1) oder unter Einschluss eines Winkels mit dieser.

17. System nach Anspruch 13, wobei sich das Urin- oder Körperflüssigkeitssammelteil (3) durch eine Öffnung (6) in dem Basisteil (1) erstreckt und wobei angrenzend an die Öffnung an der Innenseite und/oder an der Außenseite des Basisteils (1) Dichtmittel (14,17) vorgesehen sind.

18. System nach einem der vorhergehenden Ansprüche, wobei die Kapillarstruktur des Urin- oder Körperflüssigkeitssammelteils (3) ein textiles Flächengebilde in Form einer einzelnen Lage, einer doppelt oder mehrfach gefalteten Lage oder ein Material mit vergleichbaren Eigenschaften mit einem oder mehreren Endabschnitten (7) umfasst.

19. System nach einem der vorhergehenden Ansprüche, wobei die Kapillarstruktur des Urin- oder Körperflüssigkeitssammelteils (3) eines der folgenden Materialien umfasst: synthetische Garne, Multifilamentgarne, Fasern oder ein poröses Material mit Zellstruktur.

## Revendications

1. Système permettant de collecter des échantillons d'urine ou de fluides corporels exsudant de la peau d'un utilisateur, comprenant une partie de base (1) faite d'un matériau pliable adapté pour être drapé sur la peau d'un utilisateur en suivant de près les contours d'une partie de corps prédéterminée de l'utilisateur et ayant une face intérieure tournée vers la peau de l'utilisateur, et une partie de collecte d'urine ou de fluide (3) placée sur la face intérieure de la partie de base et comprenant une structure capillaire (3) qui est au moins en partie fermée par un emballage ou sac en film polymère (4) adapté pour recevoir et contenir l'urine ou les fluides excrétés par un utilisateur,
dans lequel la partie de base, sur sa face intérieure, comprend au moins une zone (10) définie par au moins une deuxième ligne ou rebord formant barrière (9) encerclant au moins une partie de la partie de collecte d'urine ou de fluide (3) exposée à un utilisateur, et au moins une première ligne ou rebord formant barrière (8) sur sa circonférence encerclant au moins une deuxième ligne ou rebord formant barrière,
et dans lequel les lignes ou rebords formant barrière sont adaptés pour établir un contact hermétique avec la peau d'un utilisateur, empêchant ainsi l'intrusion de toute contamination dans la zone (10) entourant la partie de collecte d'urine ou de fluide et la fuite d'urine ou de fluides corporels depuis celle-ci.

2. Système selon la revendication 1, dans lequel les lignes ou rebords formant barrière (9, 8) ont une forme parmi une zone surélevée, un film, une zone ou ligne imprimée ou n'importe quelle structure adaptée pour effectuer une étanchéité hermétique contre la peau d'un utilisateur.

3. Système selon la revendication 1 ou 2, dans lequel les lignes ou rebords formant barrière (9, 8) ont des caractéristiques adhésives sur une surface fournissant une adhérence efficace sur la peau d'un utilisateur et une caractéristique de décollage aisé.

4. Système selon l'une quelconque des revendications précédentes, dans lequel les lignes formant barrière (9, 8) comprennent un matériau flexible et/ou de rembourrage apte à suivre de près les contours de peau d'un utilisateur.

5. Système selon l'une quelconque des revendications précédentes, dans lequel, sur la surface de la zone de la face intérieure de la partie de base (1) entre les première et deuxième lignes ou rebords formant barrière (9, 8), des points et/ou rebords saillants sont prévus, ayant la même hauteur que les lignes ou rebords formant barrière.

6. Système selon l'une quelconque des revendications précédentes, dans lequel la zone (10) de la partie de base (1) est revêtue d'un matériau inerte non susceptible d'influer sur les caractéristiques essentielles de l'urine ou des fluides corporels reçus dans cette zone.

7. Système selon l'une quelconque des revendications précédentes, dans lequel la partie de base (1) est faite d'un matériau pliable en textile ou à base de textile.

8. Système selon la revendication 7, dans lequel le matériau en textile ou à base de textile est revêtu sur une ou ses deux faces d'un matériau inerte étanche à l'eau ou hydrofuge.

9. Système selon l'une quelconque des revendications précédentes, dans lequel la partie de base (1) est faite d'un matériau étanche à l'eau ou hydrofuge.

10. Système selon l'une quelconque des revendications précédentes, dans lequel la partie de base (1) a une forme prévue pour entourer et couvrir la zone génitale d'un utilisateur, en laissant la zone anale de l'utilisateur découverte.

11. Système selon l'une quelconque des revendications précédentes, dans lequel la partie de base (1) a des bords ou angles arrondis.

12. Système selon l'une quelconque des revendications précédentes, dans lequel la partie de base (1) a au moins une ou deux parties latéralement saillantes (2, 12).

13. Système selon l'une quelconque des revendications précédentes, dans lequel la partie de collecte d'urine ou de fluide corporel (3) s'étend d'une zone étanche à l'eau ou hydrofuge définie par barrière (10) sur la face intérieure de la partie de base (1) à une face extérieure de la partie de base et en ce qu'elle comprend au moins une partie d'extrémité intérieure (7) située dans la zone (10) et apte à recevoir de l'urine ou des fluides corporels, et une partie extérieure faisant saillie depuis la partie de base (1).

14. Système selon la revendication 13, dans lequel la partie extérieure de la partie de collecte d'urine ou de fluide corporel (3) est recouverte d'une couverture de protection faite d'un matériau opaque ou transparent (4).

15. Système selon la revendication 14, dans lequel la couverture de protection (4) est connectée de façon détachable à la partie de base (1).

16. Système selon la revendication 13, dans lequel la partie extérieure de la partie de collecte d'urine ou de fluide corporel (3) s'étend au moins partiellement sous la partie d'extrémité intérieure découverte (7) de cette dernière, soit parallèlement à un axe central de la partie de base (1), soit en contenant un angle avec celui-ci.

17. Système selon la revendication 13, dans lequel la partie de collecte d'urine ou de fluide corporel (3) s'étend à travers une ouverture (6) dans la partie de base (1) et en ce que des moyens d'étanchéité (14, 17) sont placés en position adjacente à l'ouverture sur la face intérieure et/ou la face extérieure de la partie de base (1).

18. Système selon l'une quelconque des revendications précédentes, dans lequel la structure capillaire de la partie de collecte d'urine ou de fluide corporel (3) comprend une toile ayant une forme parmi une feuille simple, une feuille pliée double ou multiple, ou un matériau ayant des caractéristiques similaires avec une ou plusieurs parties d'extrémité (7).

19. Système selon l'une quelconque des revendications précédentes, dans lequel la structure capillaire de la partie de collecte d'urine ou de fluide corporel (3) comprend un élément parmi des fils synthétiques, des multifilaments, des fibres et un matériau poreux ayant une structure cellulaire.
